# EUROPEAN PATENT APPLICATION

(11) **EP 4 644 411 A1**
(43) Date of publication of application: **05.11.2025**
(21) Application number: 24173058.9
(22) Date of filing: 29.04.2024
(51) Int. Cl.: C07K 14/72, A61K 39/00

(54) **CHIMERIC AUTOANTIGEN RECEPTOR FOR TREATING GRAVE'S DISEASE**

(71) Applicant: ISAR Bioscience GmbH, 82152 Planegg (DE)
(72) Inventor: Ammon, Tim, 82152, Planegg (DE); Hein, Rabea, 82152, Planegg (DE); Holthoff, Hans-Peter, 82152, Planegg (DE); Ungerer, Martin, 82152, Planegg (DE)
(74) Representative: Wächtershäuser & Hartz Patentanwaltspartnerschaft mbB

(57) **Abstract**

A chimeric protein comprising an extracellular domain of human thyroid-stimulating hormone receptor (TSHR), said domain lacking a C-terminal segment comprising at least the segment from amino acid residue 301 to 413 of TSHR.

## Description

### FIELD OF THE INVENTION

The present invention relates to a chimeric protein comprising an extracellular domain of a human thyroid-stimulating hormone receptor (TSHR). The chimeric protein may be a chimeric autoantigen receptor (CAAR). The invention further relates to a nucleic acid molecule encoding the chimeric protein, a vector being or comprising the nucleic acid molecule, and to a lipid nanoparticle (LNP) comprising the nucleic acid molecule or the vector. The invention further relates to a eukaryotic cell comprising the nucleic acid molecule, the vector, the LNP, and or the chimeric protein. The eukaryotic cell contains or is capable of expressing the chimeric protein, notably on its cell surface. The invention further relates to a use of the chimeric protein, the nucleic acid molecule, the vector, the LNP, or the eukaryotic cell for treating an autoimmune thyroid disease, such as Graves' disease, including Graves' orbitopathy and other Graves' disease related manifestation.

### BACKGROUND OF THE INVENTION

Graves' disease (GD, alias Morbus Basedow) is one of the most prevalent autoimmune diseases affecting 20 to 50 out of 10,000 people every year and is characterized by the presence of circulating autoantibodies that bind to and stimulate the thyroid stimulating hormone receptor (TSHR), resulting in hyperthyroidism, thyrotoxicosis and goiter (Menconi et al., 2014). Besides the aberrant activity of the thyroid gland, a large fraction of GD patients develop extrathyroidal manifestations with about 25 to 30 % of patients developing Graves' ophthalmopathy, which is characterized by persistent inflammation around the eyes, recurrent infections and exophthalmos (Weetman, 2000). In addition, GD patients may suffer from cardiovascular symptoms, such as tachyarrhythmia, and congestive heart failure, as well as Graves' dermopathy and acropachy. Although its exact etiology remains to be completely understood, GD is believed to result from a complex interaction between genetics, epigenetics, the gut microbiome, the imbalance of immune cells and environmental factors (Menconi et al., 2014; Zhou et al., 2022). Currently, antithyroid drugs (ATDs) serve as first-line treatment for GD, directly targeting the thyroid peroxidase enzyme and the production of thyroid hormones. However, half of patients relapse into hyperthyroidism upon ATD discontinuation and require more radical interventions including radioactive iodine therapy, or even, thyroidectomy (Bartalena, 2013). This underlines the unmet medical need for innovative therapeutics to specifically target the underlying cause of GD.

GD is characterized by an abnormal number of autoreactive B cells which falsely recognize thyroid epitopes on self-proteins (autoantigens) and produce antibodies that bind to the TSHR (TRAbs), overstimulating the thyroid and causing hyperthyroidism. Autoreactive B cells and secretion of autoantibodies are induced and propelled through helper T cell (Th) stimulation (Crotty, 2015). By binding to the extracellular domain of the TSHR (ecTSHR), agonistic TRAbs mimic the activity of the natural ligand TSH and thereby uncouple the production of thyroid hormones, triiodothyronine (T3) and thyroxine (T4) (Faust et al., 2022). This complex interaction cascade of stimulatory Th cells, autoreactive B cells and dysfunctional regulatory T and B cells (Treg, Breg) leads to a detrimental circle of constant autoimmune stimulation (Hansen et al., 2023).

Various biological and immunosuppressing drugs such as corticosteroids are used in the clinic to disrupt this re-stimulation circle and to partly inhibit autoantibody production. However, this may cause serious adverse effects, as pan-suppression causes an increased risk of infection (Lee and Kahaly, 2020). Furthermore, therapeutic antibodies including the anti-IGF-1 receptor antibody teprotumumab have been approved as a novel therapy, but relevant safety risks remain (Lee and Kahaly, 2020). Instead of targeting GD-related cytokine and growth factor signaling, alternative therapeutic approaches focus on immune cell ablation. Although, B cell depletion therapy with anti-CD19 and CD20-specific antibodies, respectively, has been successful in hemato-oncology, only limited efficacy was reported in GD patients (Bartalena et al., 2022).

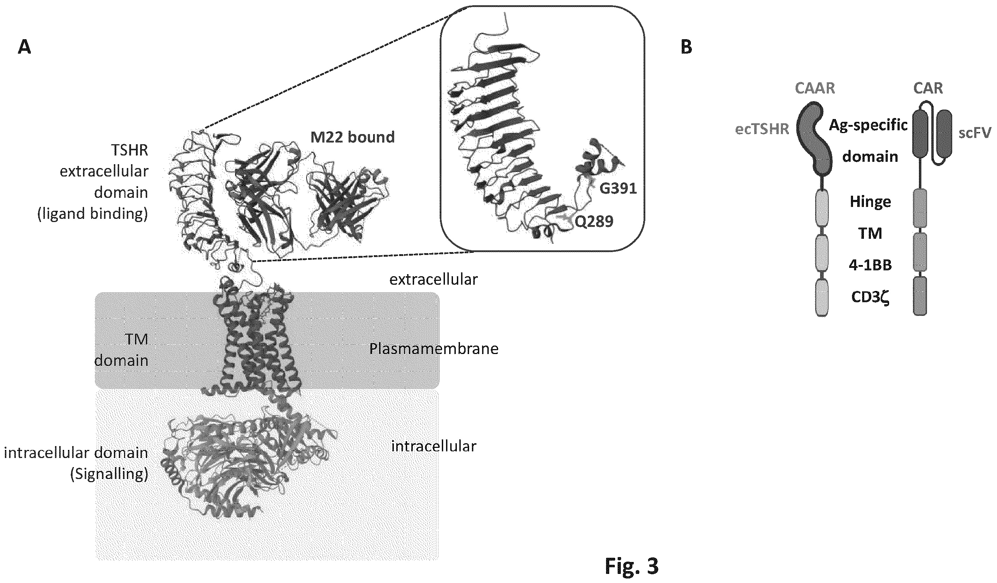

Whilst therapeutic antibodies face functional limitations in vivo such as inadequate pharmacokinetics, tissue inaccessibility and impaired interactions with the immune system, the concept of intercepting the "cellular culprits" remains attractive. Most recently, cellular immunotherapies focus on chimeric antigen receptors (CARs), genetically engineered receptors that allow specific recruitment and activation of effector immune cells towards designated antigens on target cells. For this, CARs consist of an extracellular binding domain, which determines the antigen specificity via a single chain fragment (scFv), followed by a hinge region, transmembrane domain and intracellular signaling domains (June et al., 2018) (see Fig. 3B).

As several CAR T cell therapies have proven highly effective in the context of B cell malignancies, novel targets, optimized antigen receptors and engineered immune cells have been developed to branch out to other disease areas such as cardiometabolic disorders, fibrosis, and cellular senescence (Aghajanian et al., 2022). Furthermore, first studies applied established CD19-CAR T cell products to treat autoimmune diseases, such as systemic lupus erythematosus, idiopathic inflammatory myositis, and systemic sclerosis with promising preliminary results (Müller et al., 2024). However, instead of eliminating the disease-causing autoreactive B cells, CD19-CAR T cells target almost all antibody generating B cells and their unspecific depletion might lead to significant immune deficits in patients (Hill et al., 2018).

Instead of scFv-based CARs that target the whole B cell pool, receptors termed "Chimeric Auto-Antigen Receptors" (CAARs) were considered which target the disease-causing autoreactive B cells while healthy cells should remain untouched (Nezhad *et al.,* 2020). Duan *et al.,* 2023 described a TSHR-based chimeric antigen receptor T cell that is said to deplete auto-reactive B lymphocytes for treatment of autoimmune thyroid disease. Duan *et* al. use an TSHR domain comprising aa 21-413.

In spite of the progress made in the prior art, there remains a need for better therapeutics, and tools therefor, that can be used for the treatment of autoimmune thyroid disease, such as Graves' disease. Therefore, it is an object of the present invention to provide improved means, methods, compounds and compositions that may be used for treating autoimmune thyroid disease.

### SUMMARY OF THE INVENTION

In order to solve this problem, the invention provides:
1) A chimeric protein comprising the following segments from the N-terminus to the C-terminus:
   (i) optionally a signal peptide,
   (ii) an extracellular domain of human thyroid-stimulating hormone receptor (TSHR), said domain lacking a C-terminal segment (of the ECD of human TSHR) comprising at least the segment from amino acid residue 301 to 413 of **SEQ ID NO: 1,**
   (iii) optionally a hinge region,
   (iv) a transmembrane domain, and
   (v) at least one intracellular domain.
2) The chimeric protein according to 1), wherein said protein and said extracellular domain lack a C-terminal segment comprising at least the segment from amino acid residue 295 to 413 of **SEQ ID NO: 1,** preferably said protein and said extracellular domain lack the C-terminal segment from amino acid residue 290 to 413 of **SEQ ID NO: 1.**
3) The chimeric protein according to 1) or 2), wherein said extracellular domain consists of a segment starting with amino acid residue number 30 or lower of **SEQ ID NO: 1** and extends at least to amino acid residue 280 and at most up to amino acid residue number 300 of **SEQ ID NO: 1.**
4) The chimeric protein according to any one of 1) to 3), wherein said extracellular domain consists of the segment from amino acid residue 22 to 289 of **SEQ ID NO: 1.**
5) A chimeric protein comprising the following segments from the N-terminus to the C-terminus:
   (i) optionally an N-terminal signal peptide,
   (ii) an extracellular domain of human TSHR, wherein said extracellular domain consists of a segment starting with amino acid residue number 30 or lower of **SEQ ID NO: 1** and extends at least to amino acid residue 280 and at most up to amino acid residue number 300 of **SEQ ID NO: 1,**
   (iii) optionally a hinge region,
   (iv) a transmembrane domain, and
   (v) at least one intracellular domain.
6) The chimeric protein according to 5), wherein said extracellular domain consists of a segment starting with amino acid residue number 25 or lower of **SEQ ID NO: 1** and extends at least to amino acid residue 285 and at most up to amino acid residue number 290 of **SEQ ID NO: 1.**
7) The chimeric protein according to any one of 1) to 6), wherein said hinge region is or comprises the CD8α-hinge region of **SEQ ID NO: 3** or any other hinge region derived from IgG1, IgG4 or CD28.
8) The chimeric protein according to any one of 1) to 7), wherein said transmembrane domain is the CD8α transmembrane domain of **SEQ ID NO: 5** or any other transmembrane domain derived from CD3ζ(zeta), CD4, ICOS or CD28.
9) The chimeric protein according to any one of 1) to 8), wherein said at least one intracellular domain is selected from intracellular domains of CD3ζ(zeta), CD28 IC domain, 4-1BB (CD237) IC domain, OX40 (CD134) IC domain, ICOS, CD27, MYD88-CD40, KIRD2DS2 domain, preferably is or comprises the amino acid sequence of **SEQ ID NO: 7** and/or **SEQ ID NO: 9.**
10) The chimeric protein according to any one of 1) to 9), comprising an N-terminal signal peptide, such as the CD8α-leader sequence of **SEQ ID NO: 11.**
11) The chimeric protein according to any one of 1) to 10), which is a CAAR, preferably said chimeric protein has the amino acid sequence of **SEQ ID NO: 13.**
12) The chimeric protein according to any one of 1) to 11), for use in treating an autoimmune thyroid diseases, such as Graves' disease including Graves' orbitopathy and other Graves' disease related manifestations.
13) A nucleic acid molecule encoding a chimeric protein according to any one of 1) to 11), such as the nucleic acid molecule of the nucleotide sequence of **SEQ ID NO: 14.**
14) A nucleic acid molecule comprising at least the following segments:
   (ii') a polynucleotide from nucleotide no. 34 or lower at least to nucleotide 777 of **SEQ ID NO: 16** and lacking a 3'-segment at least from nucleotide 901 to 1239 of **SEQ ID NO: 2.**
15) The nucleic acid molecule according to 13) or 14), said polynucleotide (ii') lacking a 3'-segment at least from nucleotide 883 to 1239 of **SEQ ID NO: 2,**
   preferably lacking a 3'-segment at least from nucleotide 868 to 1239 of **SEQ ID NO: 2,**
   more preferably said polynucleotide (ii') comprises a polynucleotide of **SEQ ID NO: 16.**
16) The nucleic acid molecule according to 13), 14) or 15), comprising
   (i') a polynucleotide encoding a signal peptide 5' of said polynucleotide (ii'),
   (iii') optionally a polynucleotide encoding a hinge region 3' to said polynucleotide (ii'),
   (iv') a polynucleotide encoding a transmembrane domain 3' to said polynucleotide (ii') and, if present, 3' to said polynucleotide (iii'), and
   (v') a polynucleotide encoding at least one intracellular domain.
17) The nucleic acid molecule according to any one of 13) to 16), wherein said nucleic acid molecule is a DNA (single or double-stranded) or is an RNA molecule or comprises ribonucleotides, preferably said nucleic acid molecule is an mRNA molecule.
18) A vector, such as a viral vector, being or comprising a nucleic acid molecule according to any one of 13) to 17) or encoding the chimeric protein according to any one of 1) to 12).
19) Lipid nanoparticle (LNP) comprising the nucleic acid molecule according to any one of 13) to 17) or the vector according to 18),
   preferably said nucleic acid molecule is an RNA molecule and said LNP contains said RNA molecule embedded therein.
20) The nucleic acid molecule according to any one of 13) to 17) or the vector according to 18) or the lipid nanoparticle according to 19) for use in treating an autoimmune thyroid disease, such as Graves' disease.
21) A eukaryotic cell comprising the nucleic acid molecule according any one of 13) to 17), or the vector according to 18), or the lipid nanoparticle according to 19), or the chimeric protein according to any one of 1) to 12), wherein said cell is capable of expressing said chimeric protein according to any one of 1) to 12).
22) The eukaryotic cell according to 21), wherein said nucleic acid molecule is a nuclear DNA, such as a chromosome; or wherein said eukaryotic cell comprises the nucleic acid molecule according to any one of 13) to 16) introduced via gene editing into a nuclear DNA, such as a chromosome; or wherein said nucleic acid molecule is RNA, such as mRNA.
23) The eukaryotic cell according to 17) or 18), which is a human immune cell, such as for example a T cell, a natural killer cell, a macrophage, a human induced pluripotent stem (hiPS) cell-derived cell, an autologous immune cell, or an allogenic immune cell.
24) The eukaryotic cell according to 23), wherein said immune cell is modified ex vivo or in vivo for treatment of patients.
25) The eukaryotic cell according to any one of 21) to 24), for use in treating an autoimmune thyroid disease, such as Graves' disease, such as Graves' orbitopathy and other Graves' disease related manifestations.
26) The eukaryotic cell for the use according to 25), comprising administering said cell to a patient.
27) A method of treating an autoimmune thyroid disease, such as Graves' disease, comprising administering to a patient suffering from an autoimmune thyroid disease the chimeric protein according to any one of 1) to 12), the nucleic acid molecule according to any one of 13) to 17), the vector according to 18), the LNP of 19), or the eukaryotic cell according to any one of 21) or 24).

The inventors have found a novel chimeric protein having a specific TSHR extracellular domain. The chimeric protein of the invention is a TSHR-CAAR and can be used for autoimmune cell therapy. The chimeric protein and TSHR-CAAR allows specific targeting of TSHR-autoreactive B cells and depleting autoantibodies against TSHR. The TSHR-CAAR of the invention may be used for treating GD patients. The chimeric protein or TSHR-CAAR of the invention has distinct features of the extracellular ligand binding (autoantigen) domain linked to transmembrane and signal transduction modules (see Fig. 3B). The TSHR-CAAR of the invention can have an improved expression profile and, notably, superior epitope recognition by antibodies, such as autoreactive model antibodies. Antibody binding to the TSHR-CAAR of the invention was found to be superior compared to a comparative TSHR-CAAR that is similar to a prior art TSHR-CAAR.

### BRIEF DESCRIPTION OF THE INVENTION

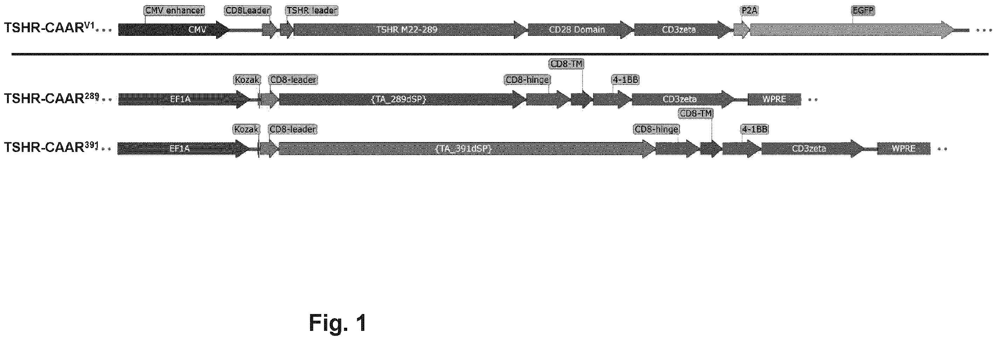

**Fig. 1****:** Schematic representation of transcription units encoded by TSHR-CAAR^{V1}, TSHR-CAAR²⁸⁹ and TSHR-CAAR³⁹¹. The genetic elements are described in section "Amino acid and nucleotide sequences" and in the Methods section below. WPRE is the Woodchuck Hepatitis Virus Posttranscriptional Regulatory Element which stabilizes expression in transgene expression.

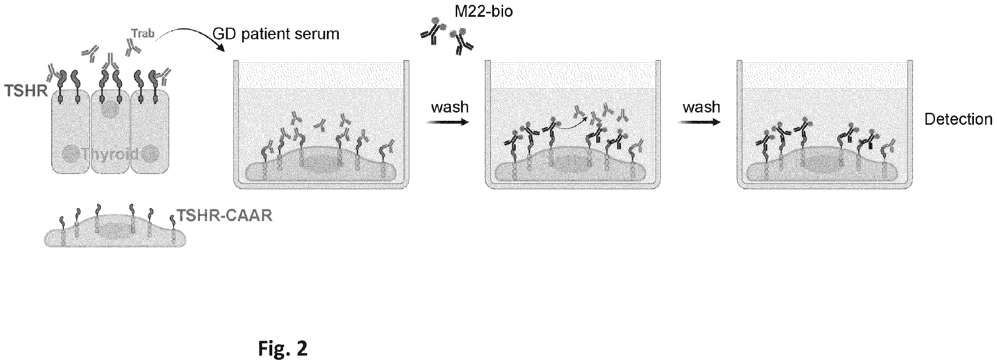

**Fig. 2****: Schematic representation of cell competitive binding assay with TSHR-CAAR and GD patient sera.** In GD patients TSH receptor-directed autoantibodies (TRAb) including M22 activate the TSHR, increase abnormal production of thyroid hormone and lead to Graves' disease and orbitopathy. For a cell competitive binding assay TSHR-CAAR expressing HEK293 cells or untreated controls were incubated with increasing dilutions of sera from GD patients or healthy donors. After serum-incubation, supernatant was removed and cells were incubated with biotinylated anti-TSHR antibody M22 (M22-bio) at 0,5 µg/mL in 100 µL OptiMEM for 1,0 h at 37 °C. Following the M22 competition step, cells were harvested, washed and stained with SA-APC for 20 minutes at 4 °C. After a final washing step, viability dye (DAPI, 1 µg/mL) was added before competition of M22 and auto-antibodies was determined via flow cytometry.
**Fig. 3** TSHR Structure and CAAR design. (A) Structural features of the TSHR (adapted from Cryo-EM structure of TSHR in complex with the activating autoantibody M22 PDB: 7XW6); Insert: highlighted amino acids in EC domain indicate terminal residues of TSHR-CAAR²⁸⁹ and TSHR-CAAR³⁹¹. (B) Schematic representation of the TSHR CAAR design compared to conventional CAR constructs including autoantigen-domain, CD8α hinge and transmembrane domain (TM), CD137 (4-1BB) and CD3zeta intracellular signaling domains.

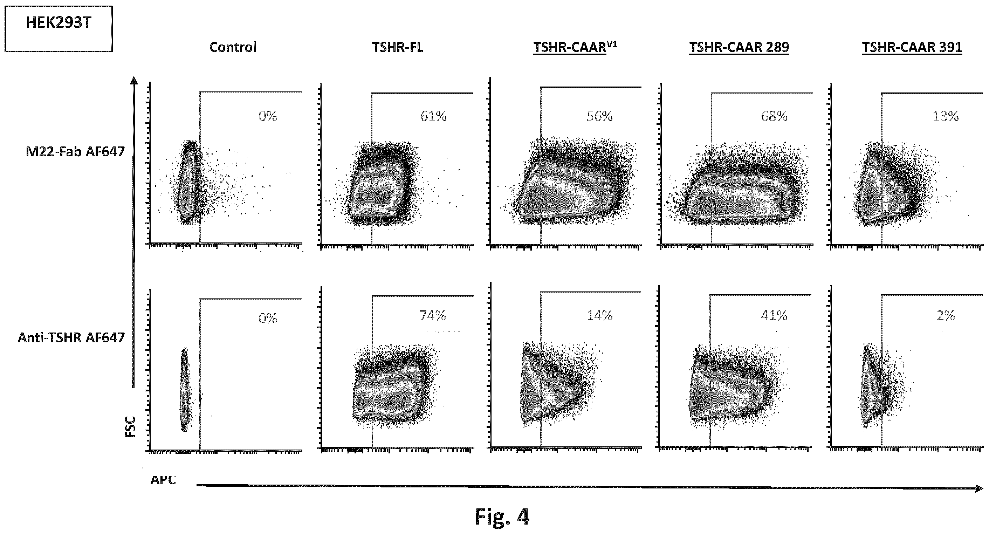

**Fig. 4** Detection of ectopic expression of TSHR and TSHR-CAAR constructs in HEK293T model cells. 48h post transfection HEK293T cells were harvested, stained with M22 (AF647-Fab coupled; upper panel) or AF647-labeled anti-THSR antibody (lower panel) and analyzed by flow cytometry. See Methods for details.

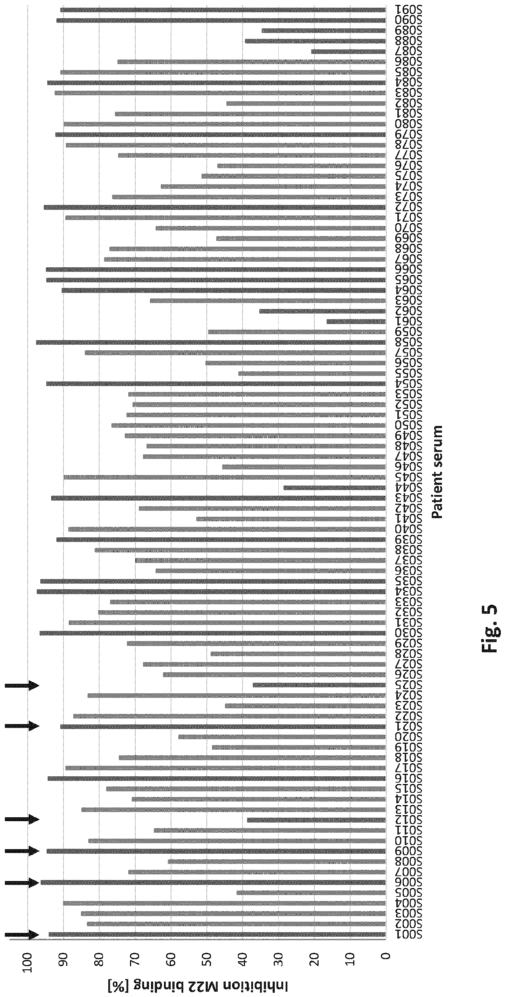

**Fig. 5** Inhibition of M22 binding by TSHR auto-antibodies in patients' serum. Level of TSHR autoantibodies was determined by commercially available competitive ELISA. Results are shown as percentage inhibition of M22 binding. Samples that show high M22 binding have high levels of anti-TSHR auto-antibodies. The arrows show the serum samples that were used for testing of binding to the TSHR-CAARs.

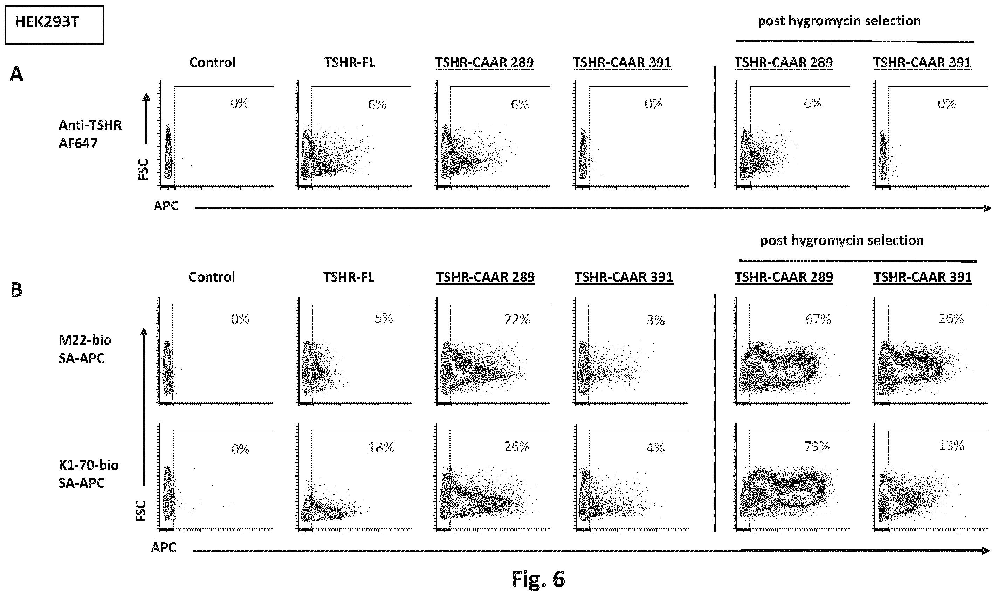

**Fig. 6** Detection of ectopic expression of TSHR-CAAR²⁸⁹ and TSHR-CAAR³⁹¹ in HEK293T model cells. (A) HEK293T cells were transfected with TSHR/TSHR-CAAR expression constructs as indicated above and stained with AF647-labeled anti-THSR antibody and analyzed by flow cytometry. (B) Analog to (A) cells were transfected and subsequently stained with M22-bio (SA-APC; upper panel) or K1-70-bio (SA-APC; lower panel). Mock-transfected cells served as negative control.

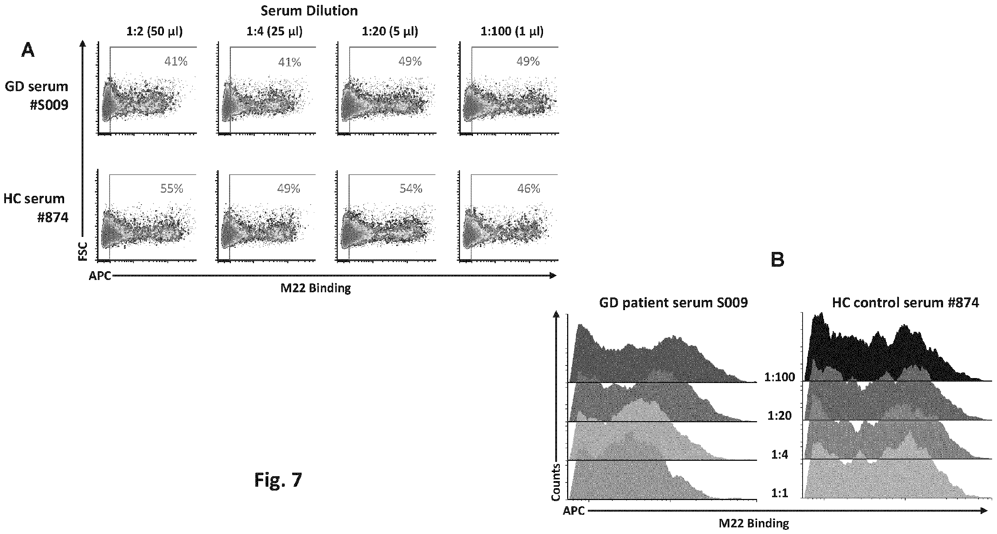

**Fig. 7****:** Flow cytometry data of cell competitive binding assay with TSHR-CAAR and GD patient serum. (A) Flow cytometry analysis of TSHR/TSHR-CAAR transfected HEK293T cells after incubation with GD patient serum or healthy control serum and subsequent M22-bio competition and streptavidin-APC stain. An example for high titer serum S009 and negative control #874 is shown. (B) Histogram overlay of M22 fluorescence data as in (A) (here sub-gated on APC-positive cells), please note the decrease in mean fluorescence intensity (MFI) on TSHR-CAAR transfected HEK293T with increasing GD patient serum, while MFI in healthy serum samples remained largely unchanged.

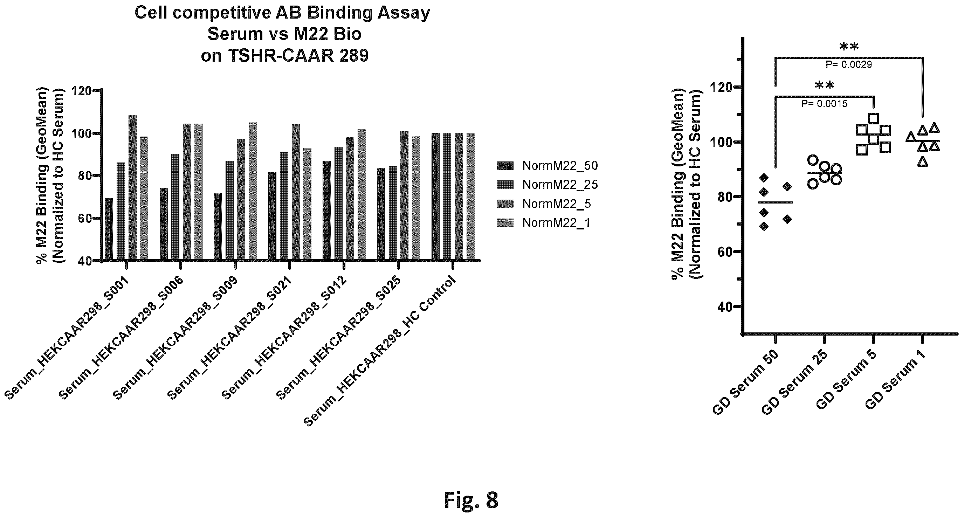

**Fig. 8** Cell competitive binding assay with TSHR-CAAR and GD patient sera. M22 binding study with GD patient and healthy donor sera, respectively, on TSHR-CAAR²⁸⁹ expressing reporter cells. MFIs of M22-APC in different serum dilutions (NormM22_50=1:2; NormM22_25=1:4; NormM22_5=1:20; NormM22_1=1:100) are normalized to healthy donor controls. GD patient serum with high antibody titers (S001; S006; S009; S021) and lower antibody titers (S012; S025) are shown. Statistical test: Kruskal-wallis: * p < 0.05; **p<0.01.

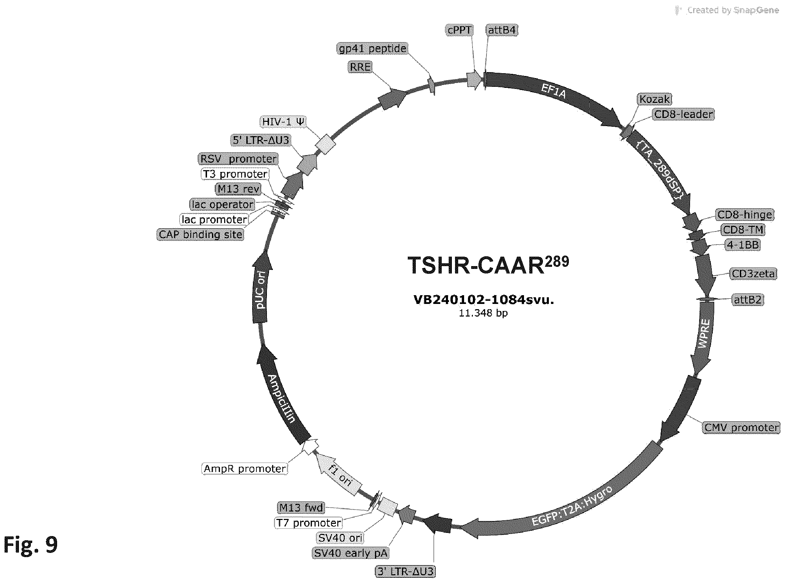

**Fig. 9** Plasmid map of TSHR-CAAR²⁸⁹. The nucleotide sequence is given in **SEQ ID NO: 17.**

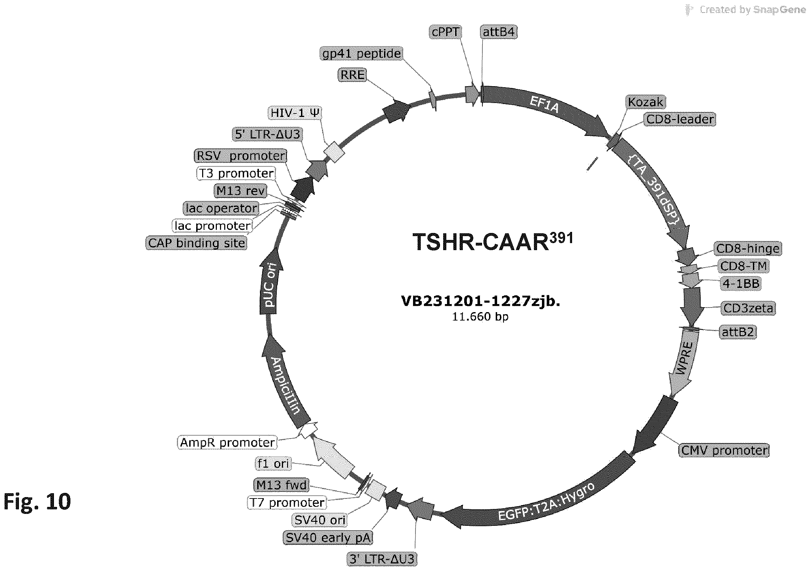

**Fig. 10** Plasmid map of TSHR-CAAR³⁹¹.

### DETAILED DESCRIPTION OF THE INVENTION

The inventors have found that a TSHR-CAAR that does not contain the entire extracellular domain (ECD) of TSHR, but lacks the loop region downstream of the LRRD domain or a major part of the loop region **(****Fig. 3****),** not only expressed better in eukaryotic cells, but showed improved antibody binding to its ligand-binding (autoimmune) domain.

The TSHR-CAAR of the invention is a chimeric protein comprising or consisting of the following segments from the N-terminus to the C-terminus:
(i) optionally a signal peptide,
(ii) an extracellular (ligand binding) domain of human TSHR, said domain lacking a (C-terminal) segment comprising (or consisting of) at least the segment from amino acid residue 301 to 413 of **SEQ ID NO: 1,**
(iii) optionally a hinge region,
(iv) a transmembrane domain, and
(v) at least one intracellular domain.

Human TSHR is synthesized as a 764 amino acid (aa) residue precursor that contains a 21 aa signal peptide, a 392 aa extracellular domain (ECD), a 269 aa membrane spanning domain consisting of seven transmembrane segments, and a 82 aa cytoplasmic domain. Herein, the amino acid numbering used starts with aa 1 of the signal peptide of human TSHR, whereby the ECD of human TSHR starts with aa 22 which is a Met residue. The amino acid sequence of the N-terminal signal peptide and ECD of human TSHR is shown in **SEQ ID NO: 1.** The ECD of human TSHR may be considered to consist of two (sub)domains, the large leucine-rich repeat domain (LRRD) and the small helical loop, both shown in the insert of **Fig. 3****.** The small helical loop is located between about aa 289 and aa 391 of **SEQ ID NO: 1;** these residues are identified in the insert of **Fig. 3****.**

In the chimeric protein of the invention, not the entire ECD of human TSHR is present, but a shorter (smaller) ECD that lacks a C-terminal segment (of the ECD of human TSHR). The (shorter) ECD used in the chimeric protein of the invention (also referred to as "the ECD of the invention") comprises the ligand binding domain of TSHR and is therefore also referred to as "extracellular ligand binding domain of human TSHR". The ECD of the invention lacks fully or partly the small helical loop shown in the insert of **Fig. 3** that is located between aa 289 and aa 391 of **SEQ ID NO: 1.** The ECD of the invention preferably lacks at least the segment from amino acid residue 301 to 413 of **SEQ ID NO: 1.** More preferably, the ECD of the invention lacks at least the segment from amino acid residue 295 to 413 of **SEQ ID NO: 1,** even more preferably it lacks at least the segment from amino acid residue 290 to 413 of **SEQ ID NO: 1.** In one embodiment of the chimeric protein, the ECD is that from aa 22 to 289 of **SEQ ID NO: 1.** It is understood that not only the ECD of the invention, but the entire chimeric protein lacks these lacking segments identified above.

The ECD of the invention preferably consists of a segment starting with amino acid residue number 30 or lower of **SEQ ID NO: 1** and extends at least to amino acid residue 280 and at most to amino acid residue number 300 of **SEQ ID NO: 1.** Preferably, the ECD consists of a segment starting with amino acid residue number 25 or lower of **SEQ ID NO: 1** and extends at least to amino acid residue 285 and at most up to amino acid residue number 290 of **SEQ ID NO: 1.** The ECD may consist of the segment from amino acid residue 22 to 289 of **SEQ ID NO: 1.** Accordingly, the chimeric protein of the invention may be defined as comprising or consisting of the following segments from the N-terminus to the C-terminus:
(i) optionally an N-terminal signal peptide,
(ii) an extracellular ligand binding domain of human TSHR, wherein said extracellular domain consists of a segment starting with amino acid residue number 30 or lower of **SEQ ID NO: 1** and extends at least to amino acid residue 280 and at most up to amino acid residue number 300 of **SEQ ID NO: 1,**
(iii) optionally a hinge region,
(iv) a transmembrane domain, and
(v) at least one intracellular domain.

The optional N-terminal signal peptide (i) is not particularly limited in the invention. Generally, it ensures, upon expressing the chimeric protein in a eukaryotic cell, signaling the chimeric protein for export and insertion into the cell membrane. An example of the signal peptide is the intrinsic signal peptide of human TSHR that may be the peptide from aa 1 to 20 of **SEQ ID NO: 1.** A preferred signal peptide is the CD8 a-!eader sequence of **SEQ ID NO: 11** that is used in the Examples. Preferably, the chimeric protein of the invention contains a signal peptide.

The chimeric protein may contain a hinge region (iii). The hinge region is not particularly limited. As the hinge region, a segment that is or comprises the CD8α-hinge region of **SEQ ID NO: 3** or any other hinge region derived from IgG1, IgG4 or CD28 may be used. Preferably, the chimeric protein contains a hinge region, more preferably it contains a signal peptide (i) and a hinge region (iii).

The chimeric protein of the invention comprises a transmembrane (TM) domain. The TM domain allows anchoring of the chimeric protein in the cell membrane, after expressing the chimeric protein in a eukaryotic cell. TM domains for such purpose are known. An example is the TM domain of human TSHR. Another example is the CD8α transmembrane domain of **SEQ ID NO: 5** that is used in the Examples. Other examples are the TM domains derived from CD3ζ(zeta), CD4, ICOS or CD28.

The chimeric protein of the invention typically comprises at least one intracellular domain (v). The function of the at least one intracellular domain is signal transduction to the interior of a cell that contains the chimeric protein in its cell membrane. The at least one intracellular domain may be selected from the intracellular domains of CD3ζ(zeta), CD28 IC domain, 4-1BB (CD237) IC domain, OX40 (CD134) IC, ICOS, CD27, MYD88-CD40, KIRD2DS2 domain, preferably is or comprises the amino acid sequence of **SEQ ID NO: 7** and/or **SEQ ID NO: 9.**

In one embodiment of the chimeric protein, the TSHR-CAAR is that of the amino acid sequence of **SEQ ID NO: 13.**

Herein, an amino acid sequence segment (or, briefly, "segment") refers to a plurality of contiguous amino acid residues of a protein or polypeptide having a larger number of amino acid residues than the segment. A protein may comprise multiple segments. Herein, different segments are non-overlapping. Protein domains may also be referred to as "segments".

The invention also provides a nucleic acid molecule encoding the chimeric protein of the invention. An example of such nucleic acid molecule is that of the nucleotide sequence of **SEQ ID NO: 14.** The invention also provides a nucleic acid molecule comprising at least the following segments:
(ii') a polynucleotide from nucleotide no. 34 or lower at least to nucleotide 777 of **SEQ ID NO: 16** and lacking a 3'-segment at least from nucleotide 901 to 1239 of **SEQ ID NO: 2;** or a polynucleotide encoding the same protein segment by the degeneracy of the genetic code.

Preferably, the polynucleotide (ii') lacks a 3'-segment at least from nucleotide 883 to 1239 of **SEQ ID NO: 2,** preferably it lacks a 3'-segment at least from nucleotide 868 to 1239 of **SEQ ID NO: 2,** more preferably said polynucleotide (ii') is or comprises a polynucleotide of **SEQ ID NO: 16.** In each of said embodiments, the invention also provides a nucleic acid molecule that encodes the same protein by the degeneracy of the genetic code.

The nucleic acid molecule generally further comprises the following segments:
(i') a polynucleotide encoding a signal peptide 5' of said polynucleotide (ii'),
(iii') optionally a polynucleotide encoding a hinge region 3' to said polynucleotide (ii'),
(iv') a polynucleotide encoding a transmembrane domain 3' to said polynucleotide (ii') and, if present, 3' to said polynucleotide (iii'), and
(v') a polynucleotide encoding at least one intracellular domain.

In the context of nucleic acids, a segment of a nucleic acid molecule refers to a plurality of contiguous nucleoside groups of a nucleic acid (molecule), the latter having a larger number of nucleoside groups than the segment. A nucleic acid molecule may comprise multiple segments. Herein, different segments are generally non-overlapping.

The nucleic acid molecule of the invention may be DNA, i.e. it may be a DNA molecule. Alternatively, the nucleic acid molecule may be RNA, i.e. it may be an RNA molecule. The RNA may be a single-stranded RNA, especially an mRNA. In some embodiments, the RNA comprises one or more modified nucleosides in place of uridine (modRNA), wherein the modified nucleoside is preferably selected from pseudouridine (ψ), N-methyl-pseudouridine (m1ψ), and 5-methyl-uridine (m5U). In some embodiments, the RNA comprises at least one of the following, preferably all of the following: a 5' cap; a 5' UTR; a 3' UTR; and a poly-A sequence. In some embodiments, the poly-A sequence comprises at least 100 A nucleotides. The 5' cap may be a cap1 or cap2 structure.

The invention further provides a vector being or comprising a nucleic acid molecule as defined above. The vector generally encodes the chimeric protein of the invention and preferably lacks a nucleic acid segment that encodes the lacking segments of the chimeric protein defined above. Apart from a polynucleotide that encodes the chimeric protein of the invention or the nucleic acid molecule defined above, the vector may further contain regulatory elements for expressing the nucleic acid in the expression system of interest and/or a selectable marker for selecting the vector in bacterial or eukaryotic cells.

The vector may be a viral vector for making use of a viral expression system. An example of a viral vector is a lentiviral vector. A lentiviral vector generally contains regulatory elements derived from a lentivirus for expressing the nucleic acid of the invention or for expressing the chimeric protein of the invention.

The invention further provides a lipid nanoparticle (LNP) comprising the nucleic acid molecule or the vector of the invention. The nucleic acid molecule is preferably an RNA molecule and said RNA molecule is embedded in the LNP. LNPs for delivering nucleic acids to human subjects have become widely known in recent years. Suitable LNPs may be those used for delivering mRNA vaccines to human subjects. Some references to documents describing such LNPs are as follows: WO2023166099 A1, Schoenmaker et al., International Journal of Pharmaceutics 601 (2021) 120586.

The invention further provides a eukaryotic cell comprising the nucleic acid molecule or the vector of the invention. Alternatively, the eukaryotic cell may comprise the chimeric protein of the invention and/or the LNP of the invention. The eukaryotic cell is preferably capable of expressing said chimeric protein. The eukaryotic cell may be a human immune cell, such as for example a T cell, a natural killer cell, a macrophage, a human induced pluripotent stem (hiPS) cell-derived cell, an autologous immune cell, or an allogenic immune cell.

The eukaryotic cell may comprise the nucleic acid molecule of the invention integrated into a nuclear DNA. In such case, this DNA may be the nucleic acid molecule of the invention. Alternatively, the eukaryotic cell may contain the nucleic acid molecule separate from a nuclear chromosome. If the nucleic acid molecule is RNA or contains ribonucleosides, the nucleic acid molecule may be contained in the cytoplasm of the cell, notably for expressing the chimeric protein from the nucleic acid molecule.

There are different ways of introducing the nucleic acid molecule into eukaryotic cells. The nucleic acid molecule may be introduced into a cell by any means that are known for this purpose, such as microinjection or lipofection. Further, cells may be transfected using LNPs containing the nucleic acid molecule, notably if the nucleic acid molecule is or contains RNA. For inserting the nucleic acid molecule into a resident DNA molecule of the cell, such as a chromosome, the gene editing technology or recombinase-mediated integration may be used.

The ultimate goal of the invention is to provide methods, means and tools (compounds) for treating an autoimmune thyroid disease, such as Graves' disease, Graves' orbitopathy and/or other Graves' disease related manifestations in a patient. There are different possibilities how the invention can be used for this purpose.

A first possibility is cell therapy using the eukaryotic cell of the invention and administering said cell to a patient, e.g. intravenously. The cell used for this purpose may be an autologous cell or an allogenic cell. For autologous cell therapy, a cell sample such as a blood sample may be obtained from the patient and the desired cells are isolated from the sample. The isolated cells may then be provided with the chimeric protein of the invention, or with the nucleic acid molecule for expressing the chimeric protein in said cell. The modified cells may then be reinserted into the patient.

In allogenic cells therapy, a suitable allogenic cell may be provided with the chimeric protein or the nucleic acid molecule similarly as described above. The modified allogenic cells may then be inserted into the patient.

For the treatment of Graves' disease, Graves' orbitopathy and/or other Graves' disease related manifestations, a preferred eukaryotic cell among those mentioned above is an immune cell, preferably a T cell or a natural killer cell. A way of introducing the nucleic acid molecule of the invention into a T cell is introducing it using gene editing into the T cell locus. After (re-)introducing the immune cell into the patient, the obtained genetically-modified immune cell may express the chimeric protein of the invention on its surface and bind autoantigenic antibodies or B cells producing such autoantigenic antibodies for treating the disease.

Accordingly, the invention provides a eukaryotic cell as described above for use in treating an autoimmune thyroid disease, such as Graves' disease, comprising administering said cell to a patient. The invention further provides a method of treating an autoimmune thyroid disease, such as Graves' disease, comprising administering to a patient suffering from an autoimmune thyroid disease the chimeric protein of the invention, the nucleic acid molecule according to the invention, the vector according to the invention, the LNP of the invention, or the eukaryotic cell according to the invention.

### EXAMPLES

### 1. Methods

### 1.1 Cell lines

The HEK293T cell line (#ACC 635; DSMZ) was used for lentiviral production. The cell lines HEK293 (#ACC 305; DSMZ) and Jurkat (#ACC305, DSMZ) were used for expression of Chimeric AutoAntigen Receptor (CAAR) constructs. HEK293T and HEK293 were cultured in Dulbecco's modified Eagle's medium (DMEM) + Glutamax supplemented with 10 % heat-inactivated fetal bovine serum (FBS), 100 U/mL penicillin, and 100 µg/mL streptomycin (all Thermo Fisher). Jurkat cells were cultivated in RPMI-1640 + Glutamax medium supplemented with 10 % FBS, 100 U/mL penicillin, 100 µg/mL streptomycin, 1 % non-essential amino acids, 1 % sodium-pyruvate and 55 µmol/L beta-mercaptoethanol (all media reagents Thermo Fisher). All cells were maintained in humidified incubators at 37 °C and 5 % CO₂.

### 1.2 Expression of TSHR-CAAR in human cells

### 1.2.1 TSHR-CAAR expression constructs

The extracellular domain of the human thyroid stimulating hormone receptor (THSR; Gene ID: 7253) was analysed and TSHR-CAAR constructs were designed and synthesized as lentiviral expression constructs (GeneArt (Thermo Fisher Scientific); Vectorbuilder). The CAAR designs are based on TSHR-derived fragments, namely AA M1-Q289 (in TSHR-CAAR^{V1}), AA M22-Q289 (in TSHR-CAAR²⁸⁹), and AA M22-G391 (in TSHR-CAAR³⁹¹) fused to the N-terminal CD8 leader as a recombinant signal peptide. While TSHR-CAAR^{V1} includes features of a typical 2^{nd} generation CAR backbone harboring CD28 and CD3zeta domains, TSHR-CAAR²⁸⁹ and TSHR-CAAR³⁹¹ contain additional co-stimulatory features of 3^{rd} generation receptors (Tomasik *et al.,* 2022): the antigen-domain is linked to a CD8α hinge and CD8 transmembrane domain to the signaling modules consisting of CD137 (4-1BB) and CD3zeta intracellular domains (see **Fig. 1**). Downstream of the TSHR CAAR expression units in TSHR-CAAR²⁸⁹ and TSHR-CAAR³⁹¹, reporters are driven by a separate cytomegaly virus (CMV) promotor with eGFP and hygromycin resistance cassettes allowing to monitor and enrich transfected/transduced target cells, while the construct TSHR-CAAR^{V1} contains a T2A peptide to link those reporter cassettes to TSHR CAAR expression.

### 1.2.2 Transfection and expression of TSHR-CAAR²⁸⁹ and TSHR-CAAR³⁹¹

Plasmid transfection was conducted using Lipofectamin 3000 (Thermo Fisher Scientific) and Mirus LT1 (Mirus Bio), respectively, according to the manufacturer's instructions. In brief, 2 µg of plasmid DNA were diluted in 125 µL OptiMEM (Thermo Fisher Scientific) and P300 reagent was added (2 µL/µg DNA). In parallel, 7.5 µL LF3000 was diluted in 125 µL OptiMEM before combining both volumes. After 15 minutes incubation at room temperature the lipofection solution was added drop-wise to HEK293(T) cells with about 80 % confluence in 6 well dishes. Selection of transfected HEK293(T) cells was achieved by culturing under hygromycin B (500 µg/mL; hygromycin B Cellpure, Roth) for at least 10 days.

### 1.2.3 Production of lentiviral particles and lentiviral transduction

For the generation of VSV-G pseudotyped lentiviral particles cargo constructs expressing TSHR-CAAR²⁸⁹ and TSHR-CAAR³⁹¹ are co-transfected with the packaging vectors psPAX and pMD2.G into 90 % confluent HEK293T cells by lipofection using Lipofectamin 3000 (Thermo Fisher Scientific). CD19-specific chimeric antigen receptor (CD19-CAR) constructs and GFP expressing vector pLV-GFPSpark served as positive controls. After 48 hours of production virus-containing culture supernatants were harvested and cell debris was removed by centrifugation and subsequent filtration through 0.8 µm cellulose acetate filters (Sartorius). Virus particles were concentrated using the Lenti-X Concentrator (Clontech Laboratories), aliquoted and frozen at -80 °C for long-term storage.

For determination of lentiviral titers, HEK293 and Jurkat cells were incubated with increasing amounts of concentrated viral particles together with 5 to 8 µg/mL Polybrene (Merck). Transduction efficacy was tested two days post transduction via flow cytometry for GFP and TSHR-CAAR expression and the multiplicity of infection (MOI) was calculated. Subsequently, target cells (Jurkat, HEK293T) were transduced with TSHR-CAAR, CD19-CAR and GFP lentiviruses at a MOI of 5 to 10. In order enhance transduction efficacy, spin-infection was performed with target cells being centrifuged at 1000 x g for 1.5 h at 30 °C.

### 1.3 TSHR Detection: Flow cytometry staining & analysis

TSHR-CAAR expression and binding of patient-derived autoantibodies was validated by flow cytometry. Therefore, cells were harvested and stained with anti-human TSHR mAbs (clone: M22 or clone: K1-70, AntibodySystem and Biorbyt). For detection, unlabeled anti-human TSHR IgGs (clone M22 or clone K1-70, AntibodySystem) were covalently linked to biotin via the EZ-Link Sulfo-NHS-LC-Biotin coupling kit (Thermo Fisher Scientific) which allows subsequent labeling via streptavidin-coupled fluorophores (Streptavidin-APC, Miltenyi Biotech). In brief, 100 µg IgG were incubated with 2.5 µL EZ-Link Sulfo-NHS-LC-Biotin (10 mM) in phosphate-buffered saline (PBS). Alternatively, M22 TSHR mAb (Biorbyt) was coupled to fluorescently labeled Fab fragments which bind to the constant Fc region (Zenon Human IgG Labeling kit; Invitrogen). For this, 5 µg M22 were incubated with 25 µL Zenon human IgG labeling reagent (here: AF647; 200 µg Fab/mL) for 5 minutes at room temperature followed by blocking free binding site with 25 µL Zenon blocking reagent (5 mg/mL) for additional 5 minutes. Fluorescent labeling of mouse anti-human K1-70 TSHR (Biorbyt) was performed using Fc-binding probes (m-IgG Fc BP-CFL 555, Santa Cruz) and 5 µg K1-70 incubated with 25 µL m-IgG Fc BP (100 µg/mL) for 15 minutes at room temperature. In addition, anti-human TSHR AF647-conjugated antibody (FAB65342R, R&D Systems) served as a positive staining control.

For flow-cytometric analysis cells were harvested and washed with PBS before incubating with human Fc block reagent (Miltenyi Biotech) in 96 wells for 10 minutes at 4 °C. After washing, cells were incubated with pre-labeled antibody staining mixes for 20 minutes at 4 °C. Biotinylated antibodies were detected by adding a second washing and incubation step with fluorophore-labeled streptavidin (SA-APC or SA-PE, Miltenyi Biotech). After a final washing step, viability dye (4',6-Diamidin-2-phenylindol (DAPI), 1 µg/mL) was added before samples were measured on a MACSQuant10 flow cytometer (Miltenyi Biotech) or a Nothern Lights 3000 flow cytometer (Cytek Biosciences, USA). Data analysis was conducted with FCS Express 7 (V. 7.20, De Novo Software).

### 1.4 Determination of anti-TSHR antibody (TRAb) level in patient sera

Sera were sampled from Graves' disease (GD) patients after informed consent and processed anonymously, as approved by the Bavarian Medical Chamber (Bayrische Ärztekammer) on September 30, 2020.

The level of anti-TSHR autoantibodies in patients' sera was determined by using the Anti-TSH Receptor (TRab) Fast ELISA (IgG) (EA 1015-9601-1 G, EUROIMMUN) according to manufacturer's instruction. The ELISA uses porcine TSH receptor and has a lower detection limit of 0.16 IU/I. In short, 75 µL serum was added per well of the antigen-coated 96-well plate, which was then incubated for 1 hour at room temperature. After a washing step, M22-peroxidase antibody was added to the wells and incubated for 25 minutes. The plate was washed three times before addition of peroxidase substrate and incubating for another 25 minutes. To terminate the reaction, stop solution was added to the wells and the color intensity was measured at 450 nm and a reference wavelength of 620 nm. The amount of TRAb was determined by calculating the percentage of inhibition of M22 binding by anti-TSHR auto-antibodies in patient sera compared to the negative control.

### 1.5 Cell competitive binding assay with TSHR-CAAR and GD patient sera

For determining the ability of the designed TSHR-CAARs to bind patient derived auto-antibodies against the TSH receptor, a competitive binding assay was performed. Therefore, TSHR-CAAR289 expressing HEK293 cells (or untreated controls, respectively) were seeded in 96 well plates (4x104 cc/well) before incubation with increasing dilutions of sera from GD patients with high or low amount of TRAb or sera from healthy donors as negative control. Sera were diluted (1:2, 1:4, 1:20 and 1:100 in OptiMEM (Thermo Fisher Scientific)) and added for 1.5 hours at 37 °C on HEK293 cells. After serum-incubation, supernatant was removed and cells were incubated with biotinylated anti-TSHR antibody M22 (M22-bio) at 0.5 µg/mL in 100 µL OptiMEM for 1 hour at 37 °C. Following the M22 competition step, cells were harvested, washed and stained with SA-APC for 20 minutes at 4 °C. After a final washing step, viability dye (DAPI, 1 µg/mL) was added before measuring. Competition of M22 and auto-antibodies was determined via flow cytometry on a MACSQuant10 flow cytometer (Miltenyi Biotech).

### 2. Results

### 2.1 Design of the TSHR-CAAR constructs

Thyroid stimulating autoantibodies cause hyperthyroidism in Graves' disease and autoantibodies exert their stimulating effect by binding to the thyroid stimulating hormone receptor (TSHR). Its extracellular domain has been identified as the dominant antigen recognized by autoantibodies in GD patients (Smith et al., 1988; Sanders et al., 2003). The structure of the TSHR, as well as the other Glycoprotein Hormone Receptors (GPHRs), contains a large amino-terminal extracellular domain (ECD) and a transmembrane domain (TMD). The TSHR ECD contains an N-terminal region, a leucine-rich repeat domain (LRRD) and a hinge region or cleavage domain. The TMD contains the typical seven transmembrane helices of GPCRs, an eighth helix parallel to the membrane and a C-terminal tail (Faust et al., 2022).

In order to specifically target TSHR-autoreactive antibody-generating B cells and their secreted immunoglobulins, parts of the extracellular domain were selected as building blocks for the design of the TSHR-CAARs. TSHR-derived LRRD-containing fragments of the ecTSHR, namely AA M1-Q289 (for TSHR-CAAR^{V1}) and the fragment AA M22-Q289 (for TSHR-CAAR²⁸⁹) lacking the intrinsic signal peptide sequence, were designed and fused to the N-terminal CD8α leader sequence. In addition to the two LRRD TSHR-CAARs mentioned above, another larger TSHR-CAAR (AA M22-G391; referred to as TSHR-CAAR³⁹¹) with an elongated ecTSHR domain was designed that is similar to a recently published construct (Duan *et al.,* 2023) and serves as "state of the art" reference construct. In construct TSHR-CAAR³⁹¹, the intrinsic signal peptide sequence is also replaced with the N-terminal CD8α leader sequence and all TSHR-autoantigen domains were combined with hinge, transmembrane and signaling domains derived from validated and published CD19-CAR constructs.

Both elongated ecTSHR constructs, TSHR-CAAR³⁹¹ and Duan CAAR (AA M22-G413), include the helical hinge loop downstream of the LRD. Structural analysis revealed that upon TSHR activation, the helical hinge contributes to the rotation of the ECD and promotes an inward movement of the extracellular tip, converting the TSHR into an inactive confirmation (Faust et al., 2022).

### 2.2 Detection of TSHR-CAAR expression on model cells

In order to validate proper expression and folding of TSHR-CAAR proteins, HEK293T cells were either transfected with TSHR-CAAR²⁸⁹, TSHR-CAAR³⁹¹, TSHR-CAAR^{V1} or full-length TSHR (TSHR-FL) and control expression constructs. After 48 hours cells were harvested and stained with the commercially available anti-TSHR antibody (FAB65342R, R&D) coupled to AF647 (**Fig. 4** lower panel). All four TSHR constructs TSHR-FL, TSHR-CAAR^{V1}, TSHR-CAAR²⁸⁹, TSHR-CAAR³⁹¹ were expressed and successfully detected by the anti-TSHR AF647 antibody with the highest signal for the full-length TSHR protein. TSHR-CAAR²⁸⁹ signal intensity was comparable to the TSHR-FL and higher than the TSHR-CAAR⁷¹. Surprisingly, the longer ECD in TSHR-CAAR³⁹¹ did show a significant weaker signal than all other constructs. Mock-transfected cells did not show any signal after staining and served as a good negative control.

In addition to protein expression, protein folding of the extracellular domains is crucial for CAAR functionality. To address this point, we made use of anti-TSH receptor antibody clones that were originally isolated from GD patients and resemble well described auto-antibodies in thyroid diseases. The M22 is a fully characterized stimulatory antibody that binds agonistically to the extracellular domain of the TSH receptor and competes with its naturally ligand thyroid stimulating hormone (TSH; **Fig. 3A**) (Furmaniak *et al.,* 2011), whereas the K1-70 is described as an inhibitory antibody for the TSH receptor. M22 binding was tested analogously to the anti-TSHR-AF647 in flow cytometry, and AF647-Fab coupled M22 robustly detected full length TSHR receptor protein as well all three TSHR-CAAR proteins (**Fig. 4** upper panel). The structural data available for the M22-ecTSHR complex highlight the non-linear antibody binding epitope buried in the six leucine-rich repeats ligand binding pocket of the TSHR. Thus, M22 binding to all three TSHR-CAAR proteins serves as a strong indicator for proper folding of the designed chimeric autoantigen domains, but also highlights high binding affinities of M22 for TSHR-CAAR²⁸⁹, while no background signal was detected in control samples.

Surprisingly, binding of M22 to the cell surface-expressed TSHR-CAAR²⁸⁹ was much stronger than to TSHR-CAAR³⁹¹ (compare the respective graphs in Fig. 4, upper panel). Thus, the engineered TSHR-CAAR²⁸⁹ molecule devoid of the helical hinge loop is much superior for antibody binding. The inventors assume that the ligand binding domain of TSHR-CAAR²⁸⁹ provides higher flexibility and accessibility of the LRRD, which could explain the superior antibody binding. Accordingly, TSHR-CAAR²⁸⁹ allows to specifically target TSHR-autoreactive antibody-generating B cells and their secreted immunoglobulins.

Based on its superior binding M22 capacities and its improved signal transduction domains compared to TSHR-CAAR^{V1}, the TSHR-CAAR²⁸⁹ construct was selected for further testing.

### 2.3 TSHR antibody level in patients' sera

Sera of GD patients were tested for anti-TSHR autoantibodies by competitive ELISA, also known as an inhibition assay. In contrast to conventional ELISAs, the assay measures the concentration of an antigen by detection of signal interference, since the sample antigen competes with a reference antigen for binding to a specific amount of labeled antibody.

For this TSHR-TRAb assay, low antibody signal represents a strong inhibition of M22 binding and therefore a high amount of TRAb in the tested serum. Samples showing an inhibition of M22 binding of over 90 % were considered having a high TRAb titer, samples with an inhibition of under 40 % were considered as having low TRAb titers. In total 90 sera were tested **(****Fig. 5****)** with 20 sera exhibiting high TRAb titers (22 %), 8 sera showed low autoantibody titers (9 %). From the samples showing high or low autoantibody titers, six sera (4 high, 2 low; see arrows **Fig. 5**) were selected for patient-derived autoantibody testing on TSHR-CAARs in a competitive FACS staining.

### 2.4 Patient-derived anti-TSHR autoantibodies (TRAb) bind to TSHR CAARs

### 2.4.1 Validation of biotinylated M22 and K1-70 in TSHR-CAAR binding assay

Measuring autoantibody binding to TSHR-CAAR proteins in a cell competitive binding assay required labeled probing antibodies, e.g. biotinylated M22 for the detection of epitope binding competition. Thus, unlabeled anti-human TSHR IgGs (clone: M22 and clone: K1-70) were covalently linked to biotin via the EZ-Link Sulfo-NHS-LC-Biotin coupling which allows subsequent labeling via streptavidin-coupled fluorophores (Streptavidin-APC, Miltenyi Biotech). These coupled antibodies were tested for TSHR and TSHR-CAAR detection by flow cytometry. HEK293T cells were either transfected with TSHR-CAAR289, TSHR-CAAR391 or full-length TSHR and control expression constructs. In addition, antibiotic selection of transfectants over 10 days allowed enrichment of hygromycin B-resistant HEK293T. Cells were harvested and stained with the biotinylated M22 (M22-bio) and K1-70 (K1-70-bio) with subsequent streptavidin-APC labeling. In parallel, staining cells with the commercially available anti-TSHR-AF647 antibody (FAB65342R, R&D) served as control. All three TSHR constructs TSHR-FL, TSHR-CAAR289, TSHR-CAAR391 were expressed and detected by the anti-TSHR-AF647 antibody **(****Fig. 6A****).** Here, the biotinylated M22 and K1-70 were tested and the modified variants were functional and efficient in staining TSHR-FL as well as TSHR-CAAR cells as determined by flow cytometry. Furthermore, both antibodies detected a pronounced fraction of TSHR-CAAR positive cells with an increased mean fluorescence intensity in hygromycin-B selected HEK293T cells **(****Fig. 6B****).** Again, the TSHR-CAAR²⁸⁹ showed higher expression and signal intensity compared to the TSHR-FL and the TSHR-CAAR³⁹¹. Mock-transfected cells did not show any signal after staining and served as a good negative control.

### 2.4.2 Cell competitive binding assay with TSHR-CAAR and GD patient sera

For determining the ability of the designed TSHR-CAAR²⁸⁹ to bind patient derived auto-antibodies against the TSH receptor, a cell competitive binding assay was performed. Therefore, TSHR-CAAR²⁸⁹ expressing HEK293 cells, or untreated controls, respectively, were incubated with increasing dilutions of sera from GD patients with high or low amount of TRAb or sera from healthy donors as negative control. Upon addition of M22-bio serum-derived GD patient autoantibodies are competing for binding to their cognate epitopes namely the TSHR-CAAR protein.

In line with our hypothesis, GD patient serum with high antibody titers (S001; S006; S009; S021) led to a pronounced inhibition of M22 binding to TSHR-CAAR²⁸⁹ and thus to a reduced APC signal on reporter cells **(****Fig. 7****).** Analogous treatment with serum of healthy donors did not affect M22 binding to TSHR-CAARs which is reflected by unaltered high antibody signals at all serum dilutions **(****Fig. 7****).**

Furthermore, GD patient serum with lower antibody titers (S012; S025) was less potent in inhibiting M22 binding to TSHR-CAAR²⁸⁹ which resulted in a marginal reduction of M22-APC signal on reporter cells **(****Fig. 8****).** Analogous treatment with serum of healthy donors did not affect M22 binding to TSHR-CAAR²⁸⁹ which is reflected by unaltered high antibody signals at all serum dilutions **(****Fig. 8****).**

Combining our initial M22 CAAR staining studies with the cell competitive binding assay, data indicated a robust binding of M22 and patient derived TRAb to the new designed TSHR-CAAR²⁸⁹.

Taken together, cell expression and competitive binding assays confirm key functional features of the TSHR-CAAR²⁸⁹ molecule. Robust protein expression in reporter cells was validated by antigen-specific binding of M22, K1-70 and anti-TSHR (FAB65342R) antibodies, that was surprisingly clearly stronger than TSHR-CAAR³⁹¹ expression. Furthermore, cell surface staining proved proper folding and transport of the TSHR-CAAR²⁸⁹ protein to the cell surface as well as improved antibody binding than to TSHR-CAAR³⁹¹. In a functional assay using GD patient-derived serum antibodies, inhibiting M22 binding to TSHR-CAAR showed the capacity of TSHR-CAAR²⁸⁹ to not only be recognized by primary disease-specific autoantibodies but also serve as a surrogate indicator for targeting autoreactive B cells in respective GD patients. Therefore, the TSHR-CAARs of the invention may be used for treating autoimmune thyroid disease, such as Graves' disease.

### Amino acid and nucleotide sequences

SEQ ID NO: 1 Amino acid sequence of N-terminal signal peptide ("TSHR leader" in Fig. 1) and extracellular (ec) domain of human TSHR (aa 1-413); in italics: ECD fragment of aa 22 to 289 used in human TSHR-CAAR²⁸⁹; underlined: aa residue 301 
MRPADLLQLV LLLDLPRDLG GMGCSSPPCE CHQEEDFRVT CKDIQRIPSL
PPSTQTLKLI ETHLRTIPSH AFSNLPNISR IYVSIDVTLQ QLESHSFYNL SKVTHIEIRN
TRNLTYIDPD ALKELPLLKF LGIFNTGLKM FPDLTKVYST DIFFILEITD NPYMTSIPVN
AFQGLCNETL TLKLYNNGFT SVQGYAFNGT KLDAVYLNKN KYLTVIDKDA
FGGVYSGPSL LDVSQTSVTA LPSKGLEHLK ELIARNTWTL KKLPLSLSFL HLTRADLSYP
SHCCAFKNQK KIRGILESLM CNESSMQSLR QRKSVNALNS PLHQEYEENL
GDSIVGYKEK SKFQDTHNNA HYYVFFEEQE DEIIGFGQEL KNPQEETLQA
FDSHYDYTIC GDSEDMVCTP KSDEFNPCED IMG*
**SEQ** ID NO: 2 Coding sequence of the amino acid sequence of SEQ ID NO: 1 
atg agg ccg gcg gac ttg ctg cag ctg gtg ctg ctg ctc gac ctg ccc agg gac ctg ggc gga atg ggg
tgt tcg tct cca ccc tgc gag tgc cat cag gag gag gac ttc aga gtc acc tgc aag gat att caa cgc
atc ccc agc tta ccg ccc agt acg cag act ctg aag ctt att gag act cac ctg aga act att cca agt cat
gca ttt tct aat ctg ccc aat att tcc aga atc tac gta tct ata gat gtg act ctg cag cag ctg gaa tca
cac tcc ttc tac aat ttg agt aaa gtg act cac ata gaa att cgg aat acc agg aac tta act tac ata gac
cct gat gcc ctc aaa gag ctc ccc ctc cta aag ttc ctt ggc att ttc aac act gga ctt aaa atg ttc cct
gac ctg acc aaa gtt tat tcc act gat ata ttc ttt ata ctt gaa att aca gac aac cct tac atg acg tca
atc cct gtg aat gct ttt cag gga cta tgc aat gaa acc ttg aca ctg aag ctg tac aac aat ggc ttt act
tca gtc caa gga tat gct ttc aat ggg aca aag ctg gat gct gtt tac cta aac aag aat aaa tac ctg
aca gtt att gac aaa gat gca ttt gga gga gta tac agt gga cca agc ttg ctg gac gtg tct caa acc
agt gtc act gcc ctt cca tcc aaa ggc ctg gag cac ctg aag gaa ctg ata gca aga aac acc tgg act
ctt aag aaa ctt cca ctt tcc ttg agt ttc ctt cac ctc aca cgg gct gac ctt tct tac cca agc cac tgc tgt
gct ttt aag aat cag aag aaa atc aga gga atc ctt gag tcc ttg atg tgt aat gag agc agt atg cag
agc ttg cgc cag aga aaa tct gtg aat gcc ttg aat agc ccc ctc cac cag gaa tat gaa gag aat ctg
ggt gac agc att gtt ggg tac aag gaa aag tcc aag ttc cag gat act cat aac aac gct cat tat tac gtc
ttc ttt gaa gaa caa gag gat gag atc att ggt ttt ggc cag gag ctc aaa aac ccc cag gaa gag act
cta caa gct ttt gac agc cat tat gac tac acc ata tgt ggg gac agt gaa gac atg gtg tgt acc ccc
aag tcc gat gag ttc aac ccg tgt gaa gac ata atg ggc
**SEQ ID NO: 3** Amino acid sequence of the hinge region ("CD8α-hinge" in Fig. 1): CD8 AA A290-D337
   A KPTTTPAPRP PTPAPTIASQ PLSLRPEACR PAAGGAVHTR GLDFACD
**SEQ ID NO: 4** Coding sequence of the amino acid sequence of SEQ ID NO: 3 
gcg aag ccc acc acg acg cca gcg ccg cga cca cca aca ccg gcg ccc acc atc gcg tcg cag ccc
ctg tcc ctg cgc cca gag gcg tgc cgg cca gcg gcg ggg ggc gca gtg cac acg agg ggg ctg gac
ttc gcc tgt gat
**SEQ ID NO: 5** Amino acid sequence of the transmembrane region ("CD8-TM" in Fig. 1): CD8 AA I338-C361
   IYI WAPLAGTCGV LLLSLVITLY C
**SEQ ID NO: 6** Coding sequence of the amino acid sequence of SEQ ID NO: 5
   atc tac atc tgg gcg ccc ttg gcc ggg act tgt ggg gtc ctt etc ctg tca ctg gtt atc acc ctt tac tgc
**SEQ ID NO: 7** Amino acid sequence of the co-stimulatory domain ("4-1BB" in Fig. 1): 4-1BB AAK362-L403
   KRGRKKLLY IFKQPFMRPV QTTQEEDGCS CRFPEEEEGG CEL
**SEQ ID NO: 8** Coding sequence of the amino acid sequence of SEQ ID NO: 7 
aaa cgg ggc aga aag aaa ctc ctg tat ata ttc aaa caa cca ttt atg aga cca gta caa act act caa
gag gaa gat ggc tgt agc tgc cga ttt cca gaa gaa gaa gaa gga gga tgt gaa ctg
**SEQ ID NO: 9** Amino acid sequence of the signaling domain ("CD3zeta" in Fig. 1): CD3 zeta AA R404-R515* 
RVKFSRS ADAPAYQQGQ NQLYNELNLG RREEYDVLDK RRGRDPEMGG
KPRRKNPQEG LYNELQKDKM AEAYSEIGMK GERRRGKGHD GLYQGLSTAT
KDTYDALHMQ ALPPR
**SEQ ID NO: 10** Coding sequence of the amino acid sequence of SEQ ID NO: 9 
aga gtg aag ttc agc agg agc gca gac gcc ccc gcg tac cag cag ggc cag aac cag ctc tat aac
gag ctc aat cta gga cga aga gag gag tac gat gtt ttg gac aag aga cgt ggc cgg gac cct gag atg
ggg gga aag ccg aga agg aag aac cct cag gaa ggc ctg tac aat gaa ctg cag aaa gat aag atg
gcg gag gcc tac agt gag att ggg atg aaa ggc gag cgc cgg agg ggc aag ggg cac gat ggc ctt
tac cag ggt ctc agt aca gcc acc aag gac acc tac gac gcc ctt cac atg cag gcc ctg ccc cct cgc
**SEQ ID NO: 11** Amino acid sequence of the signal peptide ("CD8-leader" or "CD8Leader" in Fig. 1): CD8 leader AA M1-P21
   MALPVTALLLPLALLLHAARP
**SEQ ID NO: 12** Coding sequence of the amino acid sequence of SEQ ID NO: 11
   atg gcc tta cca gtg acc gcc ttg etc ctg ccg ctg gcc ttg ctg etc cac gcc gcc agg ccg
**SEQ ID NO: 13** Amino acid sequence of TSHR CAAR²⁸⁹ (see Fig. 1) of the following segments in the order shown from N- to C-terminus:
   - first 21 residues: signal peptide: CD8α-leader aa M1-P21;
   - aa 22 to 289 of human TSHR in regular script;
   - underlined: CD8α-hinge;
   - italics: transmembrane region;
   - bold: co-stimulatory domain 4-1BB AAK362-L403;
   - regular script: signaling domain: CD3zeta AA R404-R515* 
MALPVTALLL PLALLLHAAR PMGCSSPPCE CHQEEDFRVT CKDIQRIPSL PPSTQTLKLI
ETHLRTIPSH AFSNLPNISR IYVSIDVTLQ QLESHSFYNL SKVTHIEIRN TRNLTYIDPD
ALKELPLLKF LGIFNTGLKM FPDLTKVYST DIFFILEITD NPYMTSIPVN AFQGLCNETL
TLKLYNNGFT SVQGYAFNGT KLDAVYLNKN KYLTVIDKDA FGGVYSGPSL
LDVSQTSVTA LPSKGLEHLK ELIARNTWTL KKLPLSLSFL HLTRADLSYP SHCCAFKNQA
KPTTTPAPRP PTPAPTIASQ PLSLRPEACR PAAGGAVHTR GLDFACDIYI
WAPLAGTCGV LLLSLVITLY CKRGRKKLLY IFKQPFMRPV QTTQEEDGCS
CRFPEEEEGG CELRVKFSRS ADAPAYQQGQ NQLYNELNLG RREEYDVLDK
RRGRDPEMGG KPRRKNPQEG LYNELQKDKM AEAYSEIGMK GERRRGKGHD
GLYQGLSTAT KDTYDALHMQ ALPPR*
**SEQ ID NO: 14** Coding sequence of the amino acid sequence of SEQ ID NO: 13 
atg gcc tta cca gtg acc gcc ttg ctc ctg ccg ctg gcc ttg ctg ctc cac gcc gcc agg ccg atg gga tgt
agc tct cca cct tgc gag tgc cac caa gaa gag gac ttc cgg gtc aca tgc aag gac atc cag aga atc
ccc agc ctg cct cct agc aca cag acc ctg aag ctg atc gag aca cac ctg aga aca atc cct agc cac
gcc ttc tcc aac ctg cct aac atc tcc cgg atc tac gtg tcc atc gac gtg acc ctg cag cag ctg gaa tcc
cac agc ttc tac aac ctg tcc aaa gtg acc cac atc gag atc cgg aac acc cgg aac ctg acc tac atc
gac ccc gac gct ctg aaa gag ctg ccc ctg ctg aag ttc ctg ggc atc ttc aac acc ggc ctg aag atg
ttc ccc gac ctg acc aag gtg tac tct acc gac atc ttc ttc atc ctg gaa atc aca gac aac ccc tac atg
acc agc att ccc gtg aac gcc ttc cag ggc ctg tgc aac gaa acc ctg aca ctg aag ctg tac aac aac
ggc ttc acc tcc gtg cag ggc tac gcc ttc aat ggc acc aag ctg gat gcc gtg tac ctg aac aag aac
aag tac ctg acc gtg atc gac aag gac gcc ttc ggc gga gtg tac tct gga cct tct ctg ctg gat gtg tcc
cag acc tct gtg acc gct ctg ccc tct aaa ggc ctg gaa cac ctg aaa gaa ctg atc gcc cgg aac aca
tgg act ctg aag aag ctg cct ctg agc ctg tcc ttc ctg cac ctg acc aga gcc gac ctg agc tac cct tct
cac tgc tgc gcc ttc aag aac cag gcg aag ccc acc acg acg cca gcg ccg cga cca cca aca ccg
gcg ccc acc atc gcg tcg cag ccc ctg tcc ctg cgc cca gag gcg tgc cgg cca gcg gcg ggg ggc
gca gtg cac acg agg ggg ctg gac ttc gcc tgt gat atc tac atc tgg gcg ccc ttg gcc ggg act tgt
ggg gtc ctt ctc ctg tca ctg gtt atc acc ctt tac tgc aaa cgg ggc aga aag aaa ctc ctg tat ata ttc
aaa caa cca ttt atg aga cca gta caa act act caa gag gaa gat ggc tgt agc tgc cga ttt cca gaa
gaa gaa gaa gga gga tgt gaa ctg aga gtg aag ttc agc agg agc gca gac gcc ccc gcg tac cag
cag ggc cag aac cag ctc tat aac gag ctc aat cta gga cga aga gag gag tac gat gtt ttg gac aag
aga cgt ggc cgg gac cct gag atg ggg gga aag ccg aga agg aag aac cct cag gaa ggc ctg tac
aat gaa ctg cag aaa gat aag atg gcg gag gcc tac agt gag att ggg atg aaa ggc gag cgc cgg
agg ggc aag ggg cac gat ggc ctt tac cag ggt ctc agt aca gcc acc aag gac acc tac gac gcc ctt
cac atg cag gcc ctg ccc cct cgc taa
**SEQ ID NO: 15** Amino acid sequence of CAAR antigen domain: EcTSHR AA M22-Q289 
MGCSSPPCE CHQEEDFRVT CKDIQRIPSL PPSTQTLKLI ETHLRTIPSH AFSNLPNISR
IYVSIDVTLQ QLESHSFYNL SKVTHIEIRN TRNLTYIDPD ALKELPLLKF LGIFNTGLKM
FPDLTKVYST DIFFILEITD NPYMTSIPVN AFQGLCNETL TLKLYNNGFT SVQGYAFNGT
KLDAVYLNKN KYLTVIDKDA FGGVYSGPSL LDVSQTSVTA LPSKGLEHLK ELIARNTWTL
KKLPLSLSFL HLTRADLSYP SHCCAFKNQ
**SEQ ID NO: 16** Coding sequence of the amino acid sequence of SEQ ID NO: 15 
atg gga tgt agc tct cca cct tgc gag tgc cac caa gaa gag gac ttc cgg gtc aca tgc aag gac atc
cag aga atc ccc agc ctg cct cct agc aca cag acc ctg aag ctg atc gag aca cac ctg aga aca atc
cct agc cac gcc ttc tcc aac ctg cct aac atc tcc cgg atc tac gtg tcc atc gac gtg acc ctg cag cag
 
ctg gaa tcc cac agc ttc tac aac ctg tcc aaa gtg acc cac atc gag atc cgg aac acc cgg aac ctg
acc tac atc gac ccc gac gct ctg aaa gag ctg ccc ctg ctg aag ttc ctg ggc atc ttc aac acc ggc
ctg aag atg ttc ccc gac ctg acc aag gtg tac tct acc gac atc ttc ttc atc ctg gaa atc aca gac aac
ccc tac atg acc agc att ccc gtg aac gcc ttc cag ggc ctg tgc aac gaa acc ctg aca ctg aag ctg
tac aac aac ggc ttc acc tcc gtg cag ggc tac gcc ttc aat ggc acc aag ctg gat gcc gtg tac ctg
aac aag aac aag tac ctg acc gtg atc gac aag gac gcc ttc ggc gga gtg tac tct gga cct tct ctg
ctg gat gtg tcc cag acc tct gtg acc gct ctg ccc tct aaa ggc ctg gaa cac ctg aaa gaa ctg atc
gcc cgg aac aca tgg act ctg aag aag ctg cct ctg agc ctg tcc ttc ctg cac ctg acc aga gcc gac
ctg agc tac cct tct cac tgc tgc gcc ttc aag aac cag
**SEQ ID NO: 17** Nucleic acid sequence of the TSHR-CAAR²⁸⁹ plasmid

aatgtagtcttatgcaatactcttgtagtcttgcaacatggtaacgatgagttagcaacatgcctta
caaggagagaaaaagcaccgtgcatgccgattggtggaagtaaggtggtacgatcgtgccttattag
gaaggcaacagacgggtctgacatggattggacgaaccactgaattgccgcattgcagagatattgt
atttaagtgcctagctcgatacataaacgggtctctctggttagaccagatctgagcctgggagctc
tctggctaactagggaacccactgcttaagcctcaataaagcttgccttgagtgcttcaagtagtgt
gtgcccgtctgttgtgtgactctggtaactagagatccctcagacccttttagtcagtgtggaaaat
ctctagcagtggcgcccgaacagggacttgaaagcgaaagggaaaccagaggagctctctcgacgca
ggactcggcttgctgaagcgcgcacggcaagaggcgaggggcggcgactggtgagtacgccaaaaat
tttgactagcggaggctagaaggagagagatgggtgcgagagcgtcagtattaagcgggggagaatt
agatcgcgatgggaaaaaattcggttaaggccagggggaaagaaaaaatataaattaaaacatatag
tatgggcaagcagggagctagaacgattcgcagttaatcctggcctgttagaaacatcagaaggctg
tagacaaatactgggacagctacaaccatcccttcagacaggatcagaagaacttagatcattatat
aatacagtagcaaccctctattgtgtgcatcaaaggatagagataaaagacaccaaggaagcttta g
acaagatagaggaagagcaaaacaaaagtaagaccaccgcacagcaagcggccgctgatcttcagac
ctggaggaggagatatgagggacaattggagaagtgaattatataaatataaagtagtaaaaattga
accattaggagtagcacccaccaaggcaaagagaagagtggtgcagagagaaaaaagagcagtggga
ataggagctttgttccttgggttcttgggagcagcaggaagcactatgggcgcagcgtcaatgacgc
tgacggtacaggccagacaattattgtctggtatagtgcagcagcagaacaatttgctgagggctat
tgaggcgcaacagcatctgttgcaactcacagtctggggcatcaagcagctccaggcaagaatcctg
gctgtggaaagatacctaaaggatcaacagctcctggggatttggggttgctctggaaaactcattt
gcaccactgctgtgccttggaatgctagttggagtaataaatctctggaacagatttggaatcacac
gacctggatggagtgggacagagaaattaacaattacacaagcttaatacactccttaattgaagaa
tcgcaaaaccagcaagaaaagaatgaacaagaattattggaattagataaatgggcaagtttgtgga
attggtttaacataacaaattggctgtggtatataaaattattcataatgatagtaggaggcttggt
aggtttaagaatagtttttgctgtactttctatagtgaatagagttaggcagggatattcaccatta
tcgtttcagacccacctcccaaccccgaggggacccgacaggcccgaaggaatagaagaagaaggtg
gagagagagacagagacagatccattcgattagtgaacggatctcgacggtatcgctagcttttaaa
agaaaaggggggattggggggtacagtgcaggggaaagaatagtagacataatagcaacagacatac
aaactaaagaattacaaaaacaaattacaaaaattcaaaattttactagtgattatcggatcaactt
tgtatagaaaagttgggctccggtgcccgtcagtgggcagagcgcacatcgcccacagtccccgaga
agttggggggaggggtcggcaattgaaccggtgcctagagaaggtggcgcggggtaaactgggaaag
tgatgtcgtgtactggctccgcctttttcccgagggtgggggagaaccgtatataagtgcagtagtc
gccgtgaacgttctttttcgcaacgggtttgccgccagaacacaggtaagtgccgtgtgtggttccc
gcgggcctggcctctttacgggttatggcccttgcgtgccttgaattacttccacctggctgcagta
cgtgattcttgatcccgagcttcgggttggaagtgggtgggagagttcgaggccttgcgcttaagga
gccccttcgcctcgtgcttgagttgaggcctggcctgggcgctggggccgccgcgtgcgaatctggt

ggcaccttcgcgcctgtctcgctgctttcgataagtctctagccatttaaaattttgatgacctgc
tgcgacgctttttttctggcaagatagtcttgtaaatgcgggccaagatctgcacactggtatttcg
gtttttggggccgcgggcggcgacggggcccgtgcgtcccagcgcacatgttcggcgaggcggggcc
tgcgagcgcggccaccgagaatcggacgggggtagtctcaagctggccggcctgctctggtgcctgg
tctcgcgccgccgtgtatcgccccgccctgggcggcaaggctggcccggtcggcaccagttgcgtga
gcggaaagatggccgcttcccggccctgctgcagggagctcaaaatggaggacgcggcgctcgggag
agcgggcgggtgagtcacccacacaaaggaaaagggcctttccgtcctcagccgtcgcttcatgtga
ctccacggagtaccgggcgccgtccaggcacctcgattagttctcgagcttttggagtacgtcgtct
ttaggttggggggaggggttttatgcgatggagtttccccacactgagtgggtggagactgaagtta
ggccagcttggcacttgatgtaattctccttggaatttgccctttttgagtttggatcttggttcat
tctcaagcctcagacagtggttcaaagtttttttcttccatttcaggtgtcgtgacaagtttgtaca
aaaaagcaggctgccaccatggccttaccagtgaccgccttgctcctgccgctggccttgctgctcc
acgccgccaggccgatgggatgtagctctccaccttgcgagtgccaccaagaagaggacttccgggt
cacatgcaaggacatccagagaatccccagcctgcctcctagcacacagaccctgaagctgatcgag
acacacctgagaacaatccctagccacgccttctccaacctgcctaacatctcccggatctacgtgt
ccatcgacgtgaccctgcagcagctggaatcccacagcttctacaacctgtccaaagtgacccacat
cgagatccggaacacccggaacctgacctacatcgaccccgacgctctgaaagagctgcccctgctg
aagttcctgggcatcttcaacaccggcctgaagatgttccccgacctgaccaaggtgtactctaccg
acatcttcttcatcctggaaatcacagacaacccctacatgaccagcattcccgtgaacgccttcca
gggcctgtgcaacgaaaccctgacactgaagctgtacaacaacggcttcacctccgtgcagggctac
gccttcaatggcaccaagctggatgccgtgtacctgaacaagaacaagtacctgaccgtgatcgaca
aggacgccttcggcggagtgtactctggaccttctctgctggatgtgtcccagacctctgtgaccgc
tctgccctctaaaggcctggaacacctgaaagaactgatcgcccggaacacatggactctgaagaag
ctgcctctgagcctgtccttcctgcacctgaccagagccgacctgagctacccttctcactgctgcg
ccttcaagaaccaggcgaagcccaccacgacgccagcgccgcgaccaccaacaccggcgcccaccat
cgcgtcgcagcccctgtccctgcgcccagaggcgtgccggccagcggcggggggcgcagtgcacacg
agggggctggacttcgcctgtgatatctacatctgggcgcccttggccgggacttgtggggtccttc
tcctgtcactggttatcaccctttactgcaaacggggcagaaagaaactcctgtatatattcaaaca
accatttatgagaccagtacaaactactcaagaggaagatggctgtagctgccgatttccagaagaa
gaagaaggaggatgtgaactgagagtgaagttcagcaggagcgcagacgcccccgcgtaccagcagg
gccagaaccagctctataacgagctcaatctaggacgaagagaggagtacgatgttttggacaagag
acgtggccgggaccctgagatggggggaaagccgagaaggaagaaccctcaggaaggcctgtacaat
gaactgcagaaagataagatggcggaggcctacagtgagattgggatgaaaggcgagcgccggaggg
gcaaggggcacgatggcctttaccagggtctcagtacagccaccaaggacacctacgacgcccttca
catgcaggccctgccccctcgctaaacccagctttcttgtacaaagtggtgataatcgaattccgat
aatcaacctctggattacaaaatttgtgaaagattgactggtattcttaactatgttgctcctttta
cgctatgtggatacgctgctttaatgcctttgtatcatgctattgcttcccgtatggctttcatttt
ctcctccttgtataaatcctggttgctgtctctttatgaggagttgtggcccgttgtcaggcaacgt
ggcgtggtgtgcactgtgtttgctgacgcaacccccactggttggggcattgccaccacctgtcagc
tcctttccgggactttcgctttccccctccctattgccacggcggaactcatcgccgcctgccttgc
ccgctgctggacaggggctcggctgttgggcactgacaattccgtggtgttgtcggggaagctgacg
tcctttccatggctgctcgcctgtgttgccacctggattctgcgcgggacgtccttctgctacgtcc
cttcggccctcaatccagcggaccttccttcccgcggcctgctgccggctctgcggcctcttccgcg
tcttcgccttcgccctcagacgagtcggatctccctttgggccgcctccccgcatcgggaattcccg
cggttcgaacgcgttgacattgattattgactagttattaatagtaatcaattacggggtcattagt
tcatagcccatatatggagttccgcgttacataacttacggtaaatggcccgcctggctgaccgccc
aacgacccccgcccattgacgtcaataatgacgtatgttcccatagtaacgccaatagggactttcc
attgacgtcaatgggtggagtatttacggtaaactgcccacttggcagtacatcaagtgtatcatat

gccaagtacgccccctattgacgtcaatgacggtaaatggcccgcctggcattatgcccagtacatg
accttatgggactttcctacttggcagtacatctacgtattagtcatcgctattaccatggtgatgc
ggttttggcagtacatcaatgggcgtggatagcggtttgactcacggggatttccaagtctccaccc
cattgacgtcaatgggagtttgttttggcaccaaaatcaacgggactttccaaaatgtcgtaacaac
tccgccccattgacgcaaatgggcggtaggcgtgtacggtgggaggtctatataagcagagctctct
ggctaactagagaacccactgcgccaccatggtgagcaagggcgaggagctgttcaccggggtggtg
cccatcctggtcgagctggacggcgacgtaaacggccacaagttcagcgtgtccggcgagggcgagg
gcgatgccacctacggcaagctgaccctgaagttcatctgcaccaccggcaagctgcccgtgccctg
gcccaccctcgtgaccaccctgacctacggcgtgcagtgcttcagccgctaccccgaccacatgaag
cagcacgacttcttcaagtccgccatgcccgaaggctacgtccaggagcgcaccatcttcttcaagg
acgacggcaactacaagacccgcgccgaggtgaagttcgagggcgacaccctggtgaaccgcatcga
gctgaagggcatcgacttcaaggaggacggcaacatcctggggcacaagctggagtacaactacaac
agccacaacgtctatatcatggccgacaagcagaagaacggcatcaaggtgaacttcaagatccgcc
acaacatcgaggacggcagcgtgcagctcgccgaccactaccagcagaacacccccatcggcgacgg
ccccgtgctgctgcccgacaaccactacctgagcacccagtccgccctgagcaaagaccccaacgag
aagcgcgatcacatggtcctgctggagttcgtgaccgccgccgggatcactctcggcatggacgagc
tgtacaagggctccggagagggcaggggaagtcttctaacatgcggggacgtggaggaaaatcccgg
ccccatgaaaaagcctgaactcaccgcgacgtctgtcgagaagtttctgatcgaaaagttcgacagc
gtctccgacctgatgcagctctcggagggcgaagaatctcgtgctttcagcttcgatgtaggagggc
gtggatatgtcctgcgggtaaatagctgcgccgatggtttctacaaagatcgttatgtttatcggca
ctttgcatcggccgcgctcccgattccggaagtgcttgacattggggaatttagcgagagcctgacc
tattgcatctcccgccgtgcacagggtgtcacgttgcaagacctgcctgaaaccgaactgcccgctg
ttctgcagccggtcgcggaggccatggatgcgatcgctgcggccgatcttagccagacgagcgggtt
cggcccattcggaccgcaaggaatcggtcaatacactacatggcgtgatttcatatgcgcgattgct
gatccccatgtgtatcactggcaaactgtgatggacgacaccgtcagtgcgtccgtcgcgcaggctc
tcgatgagctgatgctttgggccgaggactgccccgaagtccggcacctcgtgcacgcggatttcgg
ctccaacaatgtcctgacggacaatggccgcataacagcggtcattgactggagcgaggcgatgttc
ggggattcccaatacgaggtcgccaacatcttcttctggaggccgtggttggcttgtatggagcagc
agacgcgctacttcgagcggaggcatccggagcttgcaggatcgccgcggctccgggcgtatatgct
ccgcattggtcttgaccaactctatcagagcttggttgacggcaatttcgatgatgcagcttgggcg
cagggtcgatgcgacgcaatcgtccgatccggagccgggactgtcgggcgtacacaaatcgcccgca
gaagcgcggccgtctggaccgatggctgtgtagaagtactcgccgatagtggaaaccgacgccccag
cactcgtccgagggcaaaggaatagggtacctttaagaccaatgacttacaaggcagctgtagatct
tagccactttttaaaagaaaaggggggactggaagggctaattcactcccaacgaagacaagatctg
ctttttgcttgtactgggtctctctggttagaccagatctgagcctgggagctctctggctaactag
ggaacccactgcttaagcctcaataaagcttgccttgagtgcttcaagtagtgtgtgcccgtctgtt
gtgtgactctggtaactagagatccctcagacccttttagtcagtgtggaaaatctctagcagtagt
agttcatgtcatcttattattcagtatttataacttgcaaagaaatgaatatcagagagtgagagga
acttgtttattgcagcttataatggttacaaataaagcaatagcatcacaaatttcacaaataaagc
atttttttcactgcattctagttgtggtttgtccaaactcatcaatgtatcttatcatgtctggctc
tagctatcccgcccctaactccgcccatcccgcccctaactccgcccagttccgcccattctccgcc
ccatggctgactaattttttttatttatgcagaggccgaggccgcctcggcctctgagctattccag
aagtagtgaggaggcttttttggaggcctagggacgtacccaattcgccctatagtgagtcgtatta
cgcgcgctcactggccgtcgttttacaacgtcgtgactgggaaaaccctggcgttacccaacttaat
cgccttgcagcacatccccctttcgccagctggcgtaatagcgaagaggcccgcaccgatcgccctt
cccaacagttgcgcagcctgaatggcgaatggacgcgccctgtagcggcgcattaagcgcggcggg
tgtggtggttacgcgcagcgtgaccgctacacttgccagcgccctagcgcccgctcctttcgctttc
ttcccttcctttctcgccacgttcgccggctttccccgtcaagctctaaatcgggggctccctttag

ggttccgatttagtgctttacggcacctcgaccccaaaaaacttgattagggtgatggttcacgtag
tgggccatcgccctgatagacggtttttcgccctttgacgttggagtccacgttctttaatagtgga
ctcttgttccaaactggaacaacactcaaccctatctcggtctattcttttgatttataagggattt
tgccgatttcggcctattggttaaaaaatgagctgatttaacaaaaatttaacgcgaattttaacaa
aatattaacgcttacaatttaggtggcacttttcggggaaatgtgcgcggaacccctatttgtttat
ttttctaaatacattcaaatatgtatccgctcatgagacaataaccctgataaatgcttcaataata
ttgaaaaaggaagagtatgagtattcaacatttccgtgtcgcccttattcccttttttgcggcattt
tgccttcctgtttttgctcacccagaaacgctggtgaaagtaaaagatgctgaagatcagttgggtg
cacgagtgggttacatcgaactggatctcaacagcggtaagatccttgagagttttcgccccgaaga
acgttttccaatgatgagcacttttaaagttctgctatgtggcgcggtattatcccgtattgacgcc
gggcaagagcaactcggtcgccgcatacactattctcagaatgacttggttgagtactcaccagtca
cagaaaagcatcttacggatggcatgacagtaagagaattatgcagtgctgccataaccatgagtga
taacactgcggccaacttacttctgacaacgatcggaggaccgaaggagctaaccgcttttttgcac
aacatgggggatcatgtaactcgccttgatcgttgggaaccggagctgaatgaagccataccaaacg
acgagcgtgacaccacgatgcctgtagcaatggcaacaacgttgcgcaaactattaactggcgaact
acttactctagcttcccggcaacaattaatagactggatggaggcggataaagttgcaggaccactt
ctgcgctcggcccttccggctggctggtttattgctgataaatctggagccggtgagcgtgggtctc
gcggtatcattgcagcactggggccagatggtaagccctcccgtatcgtagttatctacacgacggg
gagtcaggcaactatggatgaacgaaatagacagatcgctgagataggtgcctcactgattaagcat
tggtaactgtcagaccaagtttactcatatatactttagattgatttaaaacttcatttttaattta
aaaggatctaggtgaagatcctttttgataatctcatgaccaaaatcccttaacgtgagttttcgtt
ccactgagcgtcagaccccgtagaaaagatcaaaggatcttcttgagatcctttttttctgcgcgta
atctgctgcttgcaaacaaaaaaaccaccgctaccagcggtggtttgtttgccggatcaagagctac
caactctttttccgaaggtaactggcttcagcagagcgcagataccaaatactgttcttctagtgta
gccgtagttaggccaccacttcaagaactctgtagcaccgcctacatacctcgctctgctaatcctg
ttaccagtggctgctgccagtggcgataagtcgtgtcttaccgggttggactcaagacgatagttac
cggataaggcgcagcggtcgggctgaacggggggttcgtgcacacagcccagcttggagcgaacgac
ctacaccgaactgagatacctacagcgtgagctatgagaaagcgccacgcttcccgaagagagaaag
gcggacaggtatccggtaagcggcagggtcggaacaggagagcgcacgagggagcttccaggggaa
acgcctggtatctttatagtcctgtcgggtttcgccacctctgacttgagcgtcgatttttgtgatg
ctcgtcaggggggcggagcctatggaaaaacgccagcaacgcggcctttttacggttcctggccttt
tgctggccttttgctcacatgttctttcctgcgttatcccctgattctgtggataaccgtattaccg
cctttgagtgagctgataccgctcgccgcagccgaacgaccgagcgcagcgagtcagtgagcgagga
agcggaagagcgcccaatacgcaaaccgcctctccccgcgcgttggccgattcattaatgcagctgg
cacgacaggtttcccgactggaaagcgggcagtgagcgcaacgcaattaatgtgagttagctcactc
attaggcaccccaggctttacactttatgcttccggctcgtatgttgtgtggaattgtgagcggata
acaatttcacacaggaaacagctatgaccatgattacgccaagcgcgcaattaaccctcactaaagg
gaacaaaagctggagctgcaagctt

### REFERENCES

Aghajanian, H., Rurik, J.G. and Epstein, J.A. (2022), "CAR-based therapies: opportunities for immuno-medicine beyond cancer", Nature Metabolism, Vol. 4 No. 2, pp. 163-169, doi: 10.1038/s42255-022-00537-5.
Bartalena, L. (2013), "Diagnosis and management of Graves disease: a global overview", Nature Reviews Endocrinology, Vol. 9 No. 12, pp. 724-734, doi: 10.1038/nrendo.2013.193.
Bartalena, L., Piantanida, E., Gallo, D., Ippolito, S. and Tanda, M.L. (2022), "Management of Graves' hyperthyroidism: present and future", Expert Review of Endocrinology & Metabolism, Vol. 17 No. 2, pp. 153-166, doi: 10.1080/17446651.2022.2052044.
Crotty, S. (2015), "A brief history of T cell help to B cells", Nature Publishing Group, Vol. 15 No. 3, pp. 185-189, doi: 10.1038/nri3803.
Duan, H., Jiang, Z., Chen, L., Bai, X., Cai, H., Yang, X. and Huang, H. (2023), "TSHR-based chimeric antigen receptor T cell specifically deplete auto-reactive B lymphocytes for treatment of autoimmune thyroid disease.", International Immunopharmacology, Vol. 124 No. PtA, p. 110873, doi: 10.1016/j.intimp.2023.110873.
Faust, B., Billesbølle, C.B., Suomivuori, C.-M., Singh, I., Zhang, K., Hoppe, N., Pinto, A.F.M., et al. (2022), "Autoantibody mimicry of hormone action at the thyrotropin receptor", Nature, Vol. 609 No. 7928, pp. 846-853, doi: 10.1038/s41586-022-05159-1.
Furmaniak, J., Sanders, J., Young, S., Kabelis, K., Sanders, P., Evans, M., Clark, J., et al. (2011), "In vivo effects of a human thyroid-stimulating monoclonal autoantibody (M22) and a human thyroid-blocking autoantibody (K1-70)", Auto-Immunity Highlights, Vol. 3 No. 1, pp. 19-25, doi: 10.1007/s13317-011-0025-9.
Hansen, M., Johnson, A., Weber, K.S. and O'Neill, K.L. (2023), "Characterizing the Interplay of Lymphocytes in Graves' Disease", International Journal of Molecular Sciences, Vol. 24 No. 7, p. 6835, doi: 10.3390/ijms24076835.
Hill, J.A., Li, D., Hay, K.A., Green, M.L., Cherian, S., Chen, X., Riddell, S.R., et al. (2018), "Infectious complications of CD19-targeted chimeric antigen receptor-modified T-cell immunotherapy", Blood, Vol. 131 No. 1, pp. 121-130, doi: 10.1182/blood-2017-07-793760.
June, C.H., O'Connor, R.S., Kawalekar, O.U., Ghassemi, S. and Milone, M.C. (2018), "CAR T cell immunotherapy for human cancer", Science, Vol. 359 No. 6382, pp. 1361-1365, doi: 10.1126/science.aar6711.
Lee, A.C.H. and Kahaly, G.J. (2020), "Novel Approaches for Immunosuppression in Graves' Hyperthyroidism and Associated Orbitopathy", European Thyroid Journal, Vol. 9 No. Suppl 1, pp. 17-30, doi: 10.1159/000508789.
Menconi, F., Marcocci, C. and Marinò, M. (2014), "Diagnosis and classification of Graves' disease", Autoimmunity Reviews, Vol. 13 No. 4-5, pp. 398-402, doi: 10.1016/j.autrev.2014.01.013.
Menconi, F., Marcocci, C. and Marinò, M. (2014), "Diagnosis and classification of Graves' disease", Autoimmunity Reviews, Vol. 13 No. 4-5, pp. 398-402, doi: 10.1016/j.autrev.2014.01.013.
Müller, F., Taubmann, J., Bucci, L., Wilhelm, A., Bergmann, C., Völkl, S., Aigner, M., et al. (2024), "CD19 CAR T-Cell Therapy in Autoimmune Disease - A Case Series with Follow-up", New England Journal of Medicine, Vol. 390 No. 8, pp. 687-700, doi: 10.1056/nejmoa2308917.
Nezhad, M.S., Seifalian, A., Bagheri, N., Yaghoubi, S., Karimi, M.H. and Adbollahpour-Alitappeh, M. (2020), "Chimeric Antigen Receptor Based Therapy as a Potential Approach in Autoimmune Diseases: How Close Are We to the Treatment?", Frontiers in Immunology, Vol. 11, p. 603237, doi: 10.3389/fimmu.2020.603237.
Tomasik, J., Jasiński, M. and Basak, G.W. (2022), "Next generations of CAR-T cells - new therapeutic opportunities in hematology?", *Frontiers in Immunology,* Vol. 13, p. 1034707, doi: 10.3389/fimmu.2022.1034707.
Weetman, A.P. (2000), "Graves' Disease", The New England Journal of Medicine, Vol. 343 No. 17, pp. 1236-1248, doi: 10.1056/nejm200010263431707.
Zhou, F., Wang, X., Wang, L., Sun, X., Tan, G., Wei, W., Zheng, G., et al. (2022), "Genetics, Epigenetics, Cellular Immunology, and Gut Microbiota: Emerging Links With Graves' Disease", Frontiers in Cell and Developmental Biology, Vol. 9, p. 794912, doi: 10.3389/fcell.2021.794912.

## Claims

1. A chimeric protein comprising the following segments from the N-terminus to the C-terminus:
(i) optionally a signal peptide,
(ii) an extracellular domain of human thyroid-stimulating hormone receptor (TSHR), said domain lacking a C-terminal segment comprising at least the segment from amino acid residue 301 to 413 of SEQ ID NO: 1,
(iii) optionally a hinge region,
(iv) a transmembrane domain, and
(v) at least one intracellular domain.

2. The chimeric protein according to claim 1, wherein said protein and said extracellular domain lack a C-terminal segment comprising at least the segment from amino acid residue 295 to 413 of **SEQ ID NO: 1,**
preferably said protein and said extracellular domain lack the C-terminal segment from amino acid residue 290 to 413 of **SEQ ID NO: 1.**

3. A chimeric protein comprising the following segments from the N-terminus to the C-terminus:
(i) optionally an N-terminal signal peptide,
(ii) an extracellular domain of human TSHR, wherein said extracellular domain consists of a segment starting with amino acid residue number 30 or lower of **SEQ ID NO: 1** and extends at least to amino acid residue 280 and at most up to amino acid residue number 300 of **SEQ ID NO: 1,**
(iii) optionally a hinge region,
(iv) a transmembrane domain, and
(v) at least one intracellular domain.

4. The chimeric protein according to any one of claims 1 to 3, wherein said hinge region is or comprises the CD8α-hinge region of **SEQ ID NO: 3** or any other hinge region derived from IgG1, IgG4 or CD28; and/or
wherein said transmembrane domain is the CD8α transmembrane domain of **SEQ ID NO: 5** or any other transmembrane domain derived from CD3ζ(zeta), CD4, ICOS or CD28, and/or
wherein said at least one intracellular domain is selected from intracellular domains of CD3ζ(zeta), CD28 IC domain, 4-1BB (CD237) IC domain, OX40 (CD134) IC, ICOS, CD27, MYD88-CD40, KIRD2DS2 domain, preferably is or comprises the amino acid sequence of **SEQ ID NO: 7** and/or **SEQ ID NO: 9.**

5. The chimeric protein according to any one of claims 1 to 4, comprising an N-terminal signal peptide, such as the CD8α-leader sequence of **SEQ ID NO: 11.**

6. The chimeric protein according to any one of claims 1 to 5, for use in treating an autoimmune thyroid diseases, such as Graves' disease including Graves' orbitopathy and other Graves' disease related manifestations.

7. A nucleic acid molecule encoding a chimeric protein according to any one of claims 1 to 6, such as the nucleic acid molecule of the nucleotide sequence of **SEQ ID NO: 14,** wherein said nucleic acid molecule may be a DNA molecule or an RNA molecule or comprises ribonucleotides, preferably said nucleic acid molecule is an mRNA molecule.

8. A vector, such as a viral vector, being or comprising a nucleic acid molecule according to claim 7 or encoding the chimeric protein according to any one of claims 1 to 6.

9. Lipid nanoparticle (LNP) comprising the nucleic acid molecule according to claim 7 or the vector according to claim 8,
preferably said nucleic acid molecule is an RNA molecule and said LNP contains said RNA molecule embedded therein.

10. The nucleic acid molecule according to claim 7 or the vector according to claim 8 or the lipid nanoparticle according to claim 9 for use in treating an autoimmune thyroid disease, such as Graves' disease.

11. A eukaryotic cell comprising the nucleic acid molecule according to claim 7, or the vector according to claim 8, or the lipid nanoparticle according to claim 9, or the chimeric protein according to any one of claims 1 to 6, wherein said cell contains or is capable of expressing said chimeric protein according to any one of claims 1 to 6.

12. The eukaryotic cell according to claim 11, wherein said nucleic acid molecule is a nuclear DNA, such as a chromosome; or wherein said eukaryotic cell comprises the nucleic acid molecule according to claim 7 introduced via gene editing into a nuclear DNA, such as a chromosome; or wherein said nucleic acid molecule is RNA, such as mRNA.

13. The eukaryotic cell according to claim 11 or 12, which is a human immune cell, such as a T cell, a natural killer cell, a macrophage, a human induced pluripotent stem (hiPS) cell-derived cell, an autologous immune cell, or an allogenic immune cell.

14. The eukaryotic cell according to any one of claims 11 to 13, for use in treating an autoimmune thyroid disease, such as Graves' disease, such as Graves' orbitopathy and other Graves' disease related manifestations.
